# EUROPÄISCHE PATENTANMELDUNG

(11) **EP 1 486 481 A2**
(43) Veröffentlichungstag der Anmeldung: **15.12.2004**
(21) Anmeldenummer: 04007093.0
(22) Anmeldetag: 24.03.2004
(51) Int. Cl.: C07C 45/50, C07C 47/02, C07C 29/141, C07C 29/16, C07C 31/125

(54) **Verfahren zur Hydroformylierung**

(30) Priorität: 25.03.2003 DE 10313319
(71) Anmelder: BASF AKTIENGESELLSCHAFT, 67056 Ludwigshafen (DE)
(72) Erfinder: Mackewitz, Thomas, 67354 Römerberg (DE); Volland, Martin, 69117 Heidelberg (DE); Paciello, Rocco, 67098 Bad Dürkheim (DE); Schäfer, Ansgar, 76137 Karlsruhe (DE); Breit, Bernhard, 79194 Gundelfingen (DE); Seiche, Wolfgang, 69120 Heidelberg (DE)
(74) Vertreter: Reitstötter, Kinzebach & Partner (GbR) Patentanwälte

(57) **Zusammenfassung**

Die vorliegende Erfindung betrifft ein Verfahren zur Hydroformylierung in Gegenwart eines Katalysators, umfassend wenigstens einen Komplex eines Metalls der VIII. Nebengruppe mit zur Dimerisierung über nichtkovalente Bindungen befähigten Monophosphorverbindungen als Liganden, solche Katalysatoren sowie deren Verwendung.

## Beschreibung

Die vorliegende Erfindung betrifft ein Verfahren zur Hydroformylierung von Olefinen in Gegenwart eines Katalysators, umfassend wenigstens einen Komplex eines Metalls der VIII. Nebengruppe mit zur Dimerisierung über nichtkovalente Bindungen befähigten Monophosphorverbindungen als Liganden, solche Katalysatoren sowie deren Verwendung.

Die Hydroformylierung oder Oxo-Synthese ist ein wichtiges großtechnisches Verfahren und dient der Herstellung von Aldehyden aus Olefinen, Kohlenmonoxid und Wasserstoff. Diese Aldehyde können gegebenenfalls im gleichen Arbeitsgang mit Wasserstoff zu den entsprechenden Oxo-Alkoholen hydriert werden. Die Reaktion selbst ist stark exotherm und läuft im Allgemeinen unter erhöhtem Druck und bei erhöhten Temperaturen in Gegenwart von Katalysatoren ab. Als Katalysatoren werden Co-, Rh-, Ir-, Ru-, Pd- oder Pt-Verbindungen bzw. -komplexe eingesetzt, die zur Aktivitäts- und/oder Selektivitätsbeeinflussung mit N- oder P-haltigen Liganden modifiziert sein können. Bei der Hydroformylierungsreaktion von Olefinen mit mehr als zwei C-Atomen kann es auf Grund der möglichen CO-Anlagerung an jedes der beiden C-Atome einer Doppelbindung zur Bildung von Gemischen isomerer Aldehyde kommen. Zusätzlich kann es beim Einsatz von Olefinen mit mindestens vier Kohlenstoffatomen auch zu einer Doppelbindungsisomerisierung kommen, d. h. zu einer Verschiebung interner Doppelbindungen auf eine terminale Position und umgekehrt.

Aufgrund der wesentlich größeren technischen Bedeutung der α-Aldehyde und insbesondere der n-Aldehyde wird eine Optimierung der Hydroformylierungskatalysatoren zur Erzielung einer möglichst hohen Hydroformylierungsaktivität bei gleichzeitig möglichst geringer Neigung zur Bildung nicht α-ständiger und insbesondere nicht n-ständiger Aldehyde angestrebt.

So besteht beispielsweise zur Herstellung von Ester-Weichmachern mit guten anwendungstechnischen Eigenschaften ein Bedarf an Weichmacheralkoholen mit etwa 6 bis 12 Kohlenstoffatomen, die zu einem geringen Grad verzweigt sind (so genannte semilineare Alkohole) und an entsprechenden Gemischen davon. Dazu zählt insbesondere 2-Propylheptanol und es enthaltende Alkoholgemische. Zu deren Herstellung kann man beispielsweise C₄-Kohlenwasserstoffgemische, die Butene oder Butene und Butane enthalten, einer Hydroformylierung und anschließender Aldolkondensation unterziehen. Beim Einsatz von Hydroformylierungskatalysatoren mit unzureichender n-Selektivität kann es dann bei der Hydroformylierung leicht zur Bildung von unerwünschten Produktaldehyden kommen, wodurch das gesamte Verfahren wirtschaftlich benachteiligt wird.

Es ist bekannt, bei der Rhodium-Niederdruck-Hydroformylierung phosphorhaltige Liganden zur Stabilisierung und/oder Aktivierung des Katalysatormetalls einzusetzen. Geeignete phosphorhaltige Liganden sind z. B. Phosphine, Phosphinite, Phosphonite, Phosphite, Phosphoramidite, Phosphole und Phosphabenzole. Die derzeit am weitesten verbreiteten Liganden sind Triarylphosphine, wie z. B. Triphenylphosphin und sulfoniertes Triphenylphosphin, da diese unter den Reaktionsbedingungen eine hinreichende Stabilität besitzen. Nachteilig an diesen Liganden ist jedoch, dass im Allgemeinen nur sehr hohe Ligandenüberschüsse zufriedenstellende Ausbeuten insbesondere an linearen Aldehyden liefern.

Es ist weiterhin bekannt, dass der Einsatz von Chelatliganden, die zwei zur Koordination befähigte Phosphoratom-haltige Gruppen aufweisen, sich vorteilhaft auf die erzielte n-Selektivität auswirkt (siehe Moulijn, van Leeuwen und van Santen, Catalysis, Bd. 79, S.199-248, Elsevier 1993). Nachteilig am Einsatz von Chelatliganden ist jedoch, dass zu deren Bereitstellung vielfach aufwendige Synthesen erforderlich sind und/oder sie nur in schlechten Ausbeuten erhalten werden. Es besteht somit weiterhin ein Bedarf an leicht zugänglichen Liganden für Hydroformylierungskatalysatoren, die eine Hydroformylierung mit hoher n-Selektivität ermöglichen.

M. Akazome et al. beschreiben in J. Org. Chem. 2000, 65, S. 6917-6921 die Synthese, Festkörperstruktur und das Aggregationsverhalten von Phosphinen, die einen 2-Pyridonring tragen. Ein Einsatz als Liganden für Übergangsmetallkomplexe wird nicht beschrieben.

G.R. Newkome und D.C. Hager beschreiben in J. Org. Chem. 1978, 43, S. 947-949 ein Verfahren zur Herstellung von Pyridyldiphenylphosphinen. Ein Einsatz als Liganden in Übergangsmetallkatalysatoren ist in diesem Dokument ebenfalls nicht beschrieben.

In der US 4,786,443 und der US 4,940,787 sind Verfahren zur Carbonylierung von acetylenisch ungesättigten Verbindungen in Gegenwart eines Palladium-Katalysators beschrieben. Als Liganden werden Phosphine eingesetzt, die wenigstens einen Hetarylrest, z. B. einen gegebenenfalls substituierten Pyridylrest, tragen. Der Einsatz von Phosphinen, die wenigstens eine zur Ausbildung nichtkovalenter Bindungen befähigte Gruppe aufweisen, als Liganden wird nicht beschrieben.

Die WO 80/01690 beschreibt einen Rhodium-Katalysator, der wenigstens einen Phosphinliganden umfasst, bei dem an das P-Atom zwei Arylgruppen und über eine Alkylenbrücke ein Heteroatom-haltiger Rest gebunden sind. Bei diesem Heteroatomhaltigen Rest kann es sich um eine Vielzahl verschiedener Reste handeln, wobei u. a. auch Carbonsäureamidgruppen-haltige Reste genannt werden. Dieses Dokument lehrt jedoch nicht den Einsatz von Liganden mit einer funktionellen Gruppe, die zur Ausbildung intermolekularer nichtkovalenter Bindungen befähigt ist. So betrifft das einzige Ausführungsbeispiel zu Carbonsäureamidgruppen-haltigen Liganden (N-2-Pyrolidinonylethyl)diphenylphosphin, welches nicht zur Ausbildung intermolekularer nichtkovalenter Bindungen zwischen den Amidgruppen befähigt ist. Die US 4,687,874 hat einen der WO 80/01690 vergleichbaren Offenbarungsgehalt.

In einer nachveröffentlichten Publikation von B. Breit und W. Seiche in J. Am. Chem. Soc. 2003, 125, 6608-6609 wird die Dimerisierung monodentater Liganden über Wasserstoffbrückenbindungen unter Ausbildung bidentater Donorliganden und deren Einsatz in Hydroformylierungskatalysatoren mit hoher Regioselektivität beschrieben.

Die unveröffentlichte deutsche Patentanmeldung 103 55 066.6 beschreibt ein Verfahren zur Herstellung chiraler Verbindungen in Gegenwart eines Katalysators, der wenigstens einen Übergangsmetallkomplex mit Liganden umfasst, die zur Ausbildung intermolekularer, nicht kovalenter Bindungen befähigte funktionelle Gruppen aufweisen.

Der vorliegenden Erfindung liegt die Aufgabe zugrunde, ein Hydroformylierungsverfahren zur Verfügung zu stellen, das sich zur Hydroformylierung von 1-Olefinen mit hoher n-Selektivität eignet. Vorzugsweise sollen darin Hydroformylierungskatalysatoren zum Einsatz kommen, deren Liganden leicht und in guten Ausbeuten herstellbar sind. Die Katalysatoren sollen eine hohe Selektivität zugunsten der Hydroformylierung gegenüber der Hydrierung aufweisen und/oder eine hohe Raum-/Zeit-Ausbeute ermöglichen. Sie sollen insbesondere auch mit geringeren Ligandüberschüssen gegenüber dem Katalysatormetall als bei aus dem Stand der Technik bekannten Katalysatoren gute Ausbeuten an linearen Aldehyden liefern.

Überraschenderweise wurde nun gefunden, dass diese Aufgabe durch den Einsatz von Monophosphorliganden gelöst wird, die zur Ausbildung intermolekularer nichtkovalenter Bindungen befähigt sind. Derartige Liganden können prinzipiell über intermolekulare nichtkovalente Bindungen dimerisieren und somit Pseudochelatkomplexe ausbilden.

Gegenstand der vorliegenden Erfindung ist daher ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Katalysators, umfassend wenigstens einen Komplex eines Metalls der VIII. Nebengruppe des Periodensystems der Elemente mit Liganden, die jeweils eine phosphoratomhaltige Gruppe und wenigstens eine zur Ausbildung intermolekularer, nichtkovalenter Bindungen befähigte funktionelle Gruppe aufweisen, wobei der Komplex über intermolekulare, nichtkovalente Bindungen dimerisierte Liganden aufweist und wobei der Abstand zwischen den Phosphoratomen der dimerisierten Liganden höchstens 5 Å beträgt.

Außerdem betrifft die vorliegende Erfindung Katalysatoren, umfassend Komplexe mit einem Metall der VIII. Nebengruppe des Periodensystems der Elemente, die Liganden umfassen, die eine Phosphoratom-haltige Gruppe und wenigstens eine zur Ausbildung intermolekularer nichtkovalenter Bindungen befähigte funktionelle Gruppe aufweisen.

Ein weiterer Gegenstand der Erfindung ist ein Verfahren zur Herstellung von 2-Propylheptanol, das die Hydroformylierung von Buten, eine Aldolkondensation der so erhaltenen Hydroformylierungsprodukte und die anschließende Hydrierung der Kondensationsprodukte umfasst, wobei als Hydroformylierungskatalysator ein Komplex eines Metalls der VIII. Nebengruppe mit den zuvor beschriebenen Liganden eingesetzt wird.

Es wurde gefunden, dass Monophosphorliganden (Liganden, die nur eine Phosphoratom-haltige Gruppe pro Molekül aufweisen), welche befähigt sind, über intermolekulare, nichtkovalente Bindungen Dimere zu bilden, bei denen der Abstand zwischen den beiden Phosphoratomen in einem Bereich liegt, wie er für Chelatliganden üblich ist, bei einem Einsatz in der Hydroformylierung eine so hohe n-Selektivität erzielen, wie sie ansonsten nur mit Chelatliganden erzielt wird. Liganden mit der Befähigung, über intermolekulare, nichtkovalente Bindungen Dimere zu bilden, werden im Rahmen dieser Erfindung auch als Pseudochelatliganden bezeichnet.

Erfindungsgemäß werden Liganden eingesetzt, die eine funktionelle Gruppe aufweisen, die zur Ausbildung intermolekularer, nichtkovalenter Bindungen befähigt ist. Vorzugsweise handelt es sich bei diesen Bindungen um Wasserstoffbrückenbindungen oder ionische Bindungen, insbesondere um Wasserstoffbrückenbindungen. Bei den funktionellen Gruppen kann es sich in einer bevorzugten Ausführung um zur Tautomerie befähigte Gruppen handeln. Die zur Ausbildung intermolekularer nichtkovalenter Bindungen befähigten funktionellen Gruppen befähigen die Liganden zur Assoziation, d. h. zur Ausbildung von Aggregaten in Form von Dimeren.

Ein Paar von funktionellen Gruppen zweier Liganden, die zur Ausbildung intermolekularer nichtkovalenter Bindungen befähigt sind, wird im Rahmen der vorliegenden Erfindung als "komplementäre funktionelle Gruppen" bezeichnet. "Komplementäre Verbindungen" sind Ligand/Ligand-Paare, die zueinander komplementäre funktionelle Gruppen aufweisen. Solche Paare sind zur Assoziation, d. h. zur Ausbildung von Aggregaten befähigt.

Vorzugsweise sind die zur Ausbildung intermolekularer nichtkovalenter Bindungen befähigten funktionellen Gruppen ausgewählt sind unter Hydroxyl-, primären, sekundären und tertiären Amino-, Thiol-, Keto-, Thioketon-, Imin-, Carbonsäureester-, Carbonsäureamid-, Amidin-, Urethan-, Harnstoff-, Sulfoxid-, Sulfoximin-, Sulfonsäureamid- und Sulfonsäureestergruppen.

Vorzugsweise handelt es sich bei diesen funktionellen Gruppen um sogenannte selbstkomplementäre funktionelle Gruppen, d. h. die Ausbildung der nichtkovalenten Bindungen erfolgt zwischen zwei gleichen funktionellen Gruppen der eingesetzten Liganden. Handelt es sich nur um eine Art von Liganden, die die Ligand/Ligand-Paare bilden, so spricht man von so genannten "Homo-Dimeren". Funktionelle Gruppen, die zur Tautomerie befähigt sind, können in den Dimeren jeweils in Form der gleichen oder als unterschiedliche Isomere (Tautomere) vorliegen. So können beispielsweise bei einer Keto-Enol-Tautomerie beide Monophosphorliganden in der Ketoform, beide in der Enolform oder einer in der Ketoform und einer in der Enolform vorliegen.

In einer weiteren geeigneten Ausführungsform werden in dem erfindungsgemäßen Verfahren wenigstens zwei verschiedene Liganden eingesetzt, die zur Ausbildung intermolekularer, nichtkovalenter Bindungen befähigte funktionelle Gruppen aufweisen. Hierbei bilden ausschließlich oder zumindest teilweise voneinander verschiedene Liganden die Ligand/Ligand-Paare (so genannte "Hetero-Dimere"). Die funktionellen Gruppen der beiden verschiedenen Liganden, die die nichtkovalente Bindung ausbilden, können gleiche oder voneinander verschiedene Gruppen sein. Funktionelle Gruppen, die zur Tautomerie befähigt sind, können in den Dimeren jeweils in Form der gleichen oder als unterschiedliche Isomere (Tautomere) vorliegen. Das Molmengenverhältnis der beiden Liganden, die das Hetero-Dimer bilden, liegt vorzugsweise im Bereich von 30:1 bis 1:30.

Der Abstand zwischen den Phosphoratomen der dimerisierten Liganden liegt vorzugsweise in einem Bereich von 2,5 bis 4,5 Å, besonders bevorzugt 3,5 bis 4,2 Å. Speziell geeignet ist ein Abstand zwischen den Phosphoratomen von 3,6 bis 4,1 Å, wie z. B. von 3,7 bis 4,0 Å.

Geeignete Verfahren zur Bestimmung, ob die eingesetzten Liganden befähigt sind, Dimere zu bilden, umfassen die Kristallstrukturanalyse, die Kernresonanzspektroskopie sowie Molecular-Modelling-Verfahren. Dabei ist es in der Regel ausreichend zur Bestimmung, die Liganden in nichtkomplexgebundener Form heranzuziehen. Dies gilt speziell für Molecular-Modelling-Verfahren. Es wurde zudem gefunden, dass sowohl durch Kristallstrukturanalyse, die am Festkörper erfolgt, als auch durch Kernresonanzspektroskopie in Lösung, als auch durch Berechnung der Struktur für die Gasphase im Allgemeinen zuverlässige Voraussagen über das Verhalten der eingesetzten Liganden unter den Hydroformylierungsbedingungen erzielt werden. So weisen Liganden, die nach den genannten Bestimmungsverfahren befähigt sind, Dimere zu bilden, in der Regel unter Hydroformylierungsbedingungen Eigenschaften auf, wie sie ansonsten nur für Chelatliganden üblich sind. Dazu zählt insbesondere die Erzielung einer hohen n-Selektivität bei der Hydroformylierung von 1-Olefinen. Des Weiteren wurde gefunden, das diese hohe n-Selektivität nicht mehr erzielt wird, wenn bei der Hydroformylierung die Ausbildung intermolekularer nichtkovalenter Bindungen zwischen den Liganden durch Zugabe von Säuren oder protischen Lösungsmitteln, wie z. B. Methanol, gestört wird.

In einer geeigneten Vorgehensweise zur Bestimmung, ob ein Ligand für das erfindungsgemäße Verfahren geeignet ist, werden zunächst mit Hilfe eines graphischen Molecular-Modeling-Programms alle möglichen H-Brücken-gebundenen Dimere des Liganden und seiner Tautomeren erzeugt. Diese Dimerstrukturen werden dann mit quantenchemischen Methoden optimiert. Vorzugsweise wird hierfür die Dichtefunktionaltheorie (DFT) eingesetzt, beispielsweise unter Verwendung des Funktionals B-P86 (A.D. Becke, Phys. Rev. A 1988, 38, 3098; J.P. Perdew, Phys. Rev. B 1986, 33, 8822; ibid 1986, 34, 7406(E)) und der Basis SV(P) (A. Schäfer, H. Horn, R. Ahlrichs, J. Chem. Phys. 1992, 97, 2571) in dem Programmpaket Turbomole (R. Ahlrichs, M. Bär, M. Häser, H. Horn, C. Kölmel, Chem. Phys. Lett. 1989, 162, 165; M. v. Arnim, R. Ahlrichs; J. Comput. Chem. 1998, 19, 1746) (erhältlich von der Universität Karlsruhe). Ein kommerziell erhältliches geeignetes Molecular-Modeling-Paket ist Gaussian 98 (M. J. Frisch, J. A. Pople et al., Gaussian 98, Revision A.5, Gaussian Inc., Pittsburgh (PA) 1998).

Als Pseudochelatligand sind nur solche geeignet, bei denen in der berechneten Dimerstruktur der Abstand der P-Atome weniger als 5 Å beträgt.

Für den Zweck der Erläuterung der vorliegenden Erfindung umfasst der Ausdruck "Alkyl" geradkettige und verzweigte Alkylgruppen. Vorzugsweise handelt es sich dabei um geradkettige oder verzweigte C₁-C₂₀-Alkyl, bevorzugterweise C₁-C₁₂-Alkyl-, besonders bevorzugt C₁-C₈-Alkyl- und ganz besonders bevorzugt C₁-C₄-Alkylgruppen. Beispiele für Alkylgruppen sind insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Ausdruck "Alkyl" umfasst auch substituierte Alkylgruppen, welche im Allgemeinen 1, 2, 3, 4 oder 5, bevorzugt 1, 2 oder 3 und besonders bevorzugt 1 Substituenten aufweisen. Diese sind vorzugsweise ausgewählt unter Cycloalkyl, Aryl, Hetaryl, Halogen, NE¹E², NE¹E²E³⁺, Carboxylat und Sulfonat. Eine bevorzugte Perfluoralkylgruppe ist Trifluormethyl.

Der Ausdruck "Alkylen" im Sinne der vorliegenden Erfindung steht für geradkettige oder verzweigte Alkandiyl-Gruppen mit 1 bis 5 Kohlenstoffatomen.

Der Ausdruck "Cycloalkyl" umfasst im Sinne der vorliegenden Erfindung unsubstituierte als auch substituierte Cycloalkylgruppen, vorzugsweise C₅-C₇-Cycloalkylgruppen, wie Cyclopentyl, Cyclohexyl oder Cycloheptyl. Diese können im Falle einer Substitution, im Allgemeinen 1, 2, 3, 4 oder 5, bevorzugt 1, 2 oder 3 und besonders bevorzugt 1 Substituenten tragen. Vorzugsweise sind diese Substituenten ausgewählt unter Alkyl, Alkoxy und Halogen.

Der Ausdruck "Heterocycloalkyl" im Sinne der vorliegenden Erfindung umfasst gesättigte, cycloaliphatische Gruppen mit im Allgemeinen 4 bis 7, vorzugsweise 5 oder 6 Ringatomen, in denen 1 oder 2 der Ringkohlenstoffatome durch Heteroatome, ausgewählt aus den Elementen Sauerstoff, Stickstoff und Schwefel, ersetzt sind und die gegebenenfalls substituiert sein können, wobei im Falle einer Substitution, diese heterocycloaliphatischen Gruppen 1, 2 oder 3, vorzugsweise 1 oder 2, besonders bevorzugt 1 Substituenten tragen können. Diese Substituenten sind vorzugsweise ausgewählt unter Alkyl, Aryl, COOR⁰, COO⁻M⁺ und NE¹E², besonders bevorzugt sind Alkylreste. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothiophenyl, Tetrahydrofuranyl, Tetrahydropyranyl, Dioxanyl genannt.

Der Ausdruck "Aryl" umfasst im Sinne der vorliegenden Erfindung unsubstituierte als auch substituierte Arylgruppen, und steht vorzugsweise für Phenyl, Tolyl, Xylyl, Mesityl, Naphthyl, Fluorenyl, Anthracenyl, Phenanthrenyl oder Naphthacenyl, besonders bevorzugt für Phenyl oder Naphthyl, wobei diese Arylgruppen im Falle einer Substitution im Allgemeinen 1, 2, 3, 4 oder 5, vorzugsweise 1, 2 oder 3 und besonders bevorzugt 1 Substituenten, ausgewählt aus den Gruppen Alkyl, Alkoxy, Carboxylat, Trifluormethyl, Sulfonat, NE¹E², Alkylen-NE¹E², Nitro, Cyano oder Halogen, tragen können. Eine bevorzugte Perfluorarylgruppe ist Pentafluorphenyl.

Der Ausdruck "Hetaryl" umfasst im Sinne der vorliegenden Erfindung unsubstituierte oder substituierte, heterocycloaromatische Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl. Diese heterocycloaromatischen Gruppen können im Falle einer Substitution im Allgemeinen 1, 2 oder 3 Substituenten, ausgewählt aus den Gruppen Alkyl, Alkoxy, Carboxylat, Sulfonat, NE¹E², Alkylen-NE¹E² oder Halogen, tragen.

Carboxylat und Sulfonat stehen im Rahmen dieser Erfindung vorzugsweise für ein Derivat einer Carbonsäurefunktion bzw. einer Sulfonsäurefunktion, insbesondere für ein Metallcarboxylat oder -sulfonat, eine Carbonsäureester- oder Sulfonsäureesterfunktion oder eine Carbonsäure- oder Sulfonsäureamidfunktion. Dazu zählen z. B. die Ester mit C₁-C₄₇-Alkanolen, wie Methanol, Ethanol, n-Propanol, Isopropanol, n-Butanol, sec.-Butanol und tert.-Butanol.

Die obigen Erläuterungen zu den Ausdrücken "Alkyl", "Cycloalkyl", "Aryl", "Heterocycloalkyl" und "Hetaryl" gelten entsprechend für die Ausdrücke "Alkoxy", "Cycloalkoxy", "Aryloxy", "Heterocycloalkoxy" und "Hetaryloxy".

Der Ausdruck "Acyl" steht im Sinne der vorliegenden Erfindung für Alkanoyl- oder Aroylgruppen mit im Allgemeinen 2 bis 11, vorzugsweise 2 bis 8 Kohlenstoffatomen, beispielsweise für die Acetyl-, Propanoyl-, Butanoyl-, Pentanoyl-, Hexanoyl-, Heptanoyl-, 2-Ethylhexanoyl-, 2-Propylheptanoyl-, Pivaloyl-, Benzoyl- oder Naphthoyl-Gruppe.

Die Gruppen NE¹E², NE⁴E⁵und NE⁷E⁸ stehen vorzugsweise für N,N-Dimethylamino, N,N-Diethylamino, N,N-Dipropylamino, N,N-Diisopropylamino, N,N-Di-n-butylamino, N,N-Di-t.-butylamino, N,N-Dicyclohexylamino oder N,N-Diphenylamino.

Halogen steht für Fluor, Chlor, Brom und lod, bevorzugt für Fluor, Chlor und Brom.

M⁺ steht für ein Kationäquivalent, d. h. für ein einwertiges Kation oder den einer positiven Einfachladung entsprechenden Anteil eines mehrwertigen Kations. Das Kation M⁺ dient lediglich als Gegenion zur Neutralisation negativ geladener Substituentengruppen, wie der COO⁻ oder der Sulfonat-Gruppe und kann im Prinzip beliebig gewählt werden. Vorzugsweise werden deshalb Alkalimetall-, insbesondere Na⁺, K⁺-, Li⁺-Ionen oder Onium-lonen, wie Ammonium-, Mono-, Di-, Tri-, Tetraalkylammonium-, Phosphonium-, Tetraalkyl-phosphonium- oder Tetraarylphosphonium-lonen verwendet.

Entsprechendes gilt für das Anionäquivalent X⁻, das lediglich als Gegenion positiv geladener Substituentengruppen, wie den Ammoniumgruppen, dient und beliebig gewählt werden kann unter einwertigen Anionen und den einer negativen Einfachladung entsprechenden Anteilen eines mehrwertigen Anions, wobei im Allgemeinen Halogenidlonen X⁻ bevorzugt sind, insbesondere Chlorid und Bromid.

Die Werte für x und y stehen für eine ganze Zahl von 1 bis 240, vorzugsweise für eine ganze Zahl von 3 bis 120.

Der Begriff "polycyclische Verbindung" umfasst im Rahmen der vorliegenden Erfindung im weitesten Sinne Verbindungen, die wenigstens zwei Ringe enthalten, unabhängig davon, wie diese Ringe verknüpft sind. Hierbei kann es sich um carbocyclische und/oder heterocyclische Ringe handeln. Die Ringe können über Einfach- oder Doppelbindungen verknüpft ("mehrkernige Verbindungen"), durch Anellierung verbunden ("kondensierte Ringsysteme") oder überbrückt ("überbrückte Ringsysteme", "Käfigverbindungen") sein. Bevorzugte polycyclische Verbindungen sind kondensierte Ringsysteme.

Kondensierte Ringsysteme können durch Anellierung verknüpfte (ankondensierte) aromatische, hydroaromatische und cyclische Verbindungen sein. Kondensierte Ringsysteme bestehen aus zwei, drei oder mehr als drei Ringen. Je nach der Verknüpfungsart unterscheidet man bei kondensierten Ringsystemen zwischen einer ortho-Anellierung, d. h. jeder Ring hat mit jedem Nachbarring jeweils eine Kante, bzw. zwei Atome gemeinsam, und einer peri-Anellierung, bei der ein Kohlenstoffatom mehr als zwei Ringen angehört. Bevorzugt unter den kondensierten Ringsystemen sind orthokondensierte Ringsysteme.

Die erfindungsgemäß eingesetzten Ligand/Ligand-Paare lassen sich schematisch folgendermaßen darstellen: 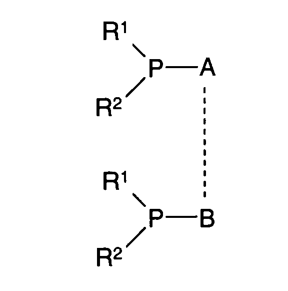 worin
- A und B: für Reste mit zueinander komplementären funktionellen Gruppen stehen, zwischen denen eine nichtkovalente Wechselwirkung besteht,
- R¹ und R²: wie im Folgenden definiert sind.

Die phosphoratomhaltige Gruppe ist vorzugsweise ausgewählt unter Gruppen der allgemeinen Formel 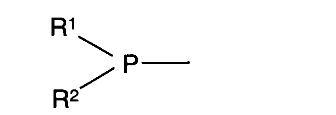 worin
- R¹ und R²: unabhängig voneinander für Alkyl, Alkoxy, Cycloalkyl, Cycloalkoxy, Heterocycloalkyl, Heterocycloalkoxy, Aryl, Aryloxy, Hetaryl oder Hetaryloxy stehen oder
- R¹ und R²: zusammen mit dem Phosphoratom, an das sie gebunden sind, für einen 5-bis 8-gliedrigen Heterocyclus stehen, der gegebenenfalls zusätzlich ein-, zwei-oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, COOR^{c}, COO⁻M⁺, SO₃R^{c}, SO₃⁻M⁺, PO₃(R^{c})(R^{d}), (PO₃)²⁻(M⁺)₂, NE⁴E⁵, (NE⁴E⁵E ⁶)⁺X⁻, OR^{e}, SR^{e}, (CHR^{f}CH₂O)_{y}R^{e}, (CH₂NE⁴)_{y}R^{e}, (CH₂CH₂NE⁴)_{y}R^{e}, Halogen, Nitro, Acyl oder Cyano stehen,
worin
- R^{c} und R^{d}: jeweils gleiche oder verschiedene Reste, ausgewählt unter Alkyl, Cycloalkyl, Aryl oder Hetaryl bedeuten,
- R^{e}, E⁴, E⁵, E⁶: jeweils gleiche oder verschiedene Reste, ausgewählt unter Wasserstoff, Alkyl, Cycloalkyl, Aryl oder Hetaryl bedeuten,
- R^{f}: für Wasserstoff, Methyl oder Ethyl steht,
- M⁺: für ein Kationäquivalent steht,
- X⁻: für ein Anionäquivalent steht und
- y: für eine ganze Zahl von 1 bis 240 steht.

Nach einer ersten bevorzugten Ausführungsform sind R¹ und R² nicht miteinander verbrückt. Dann sind R¹ und R² vorzugsweise unabhängig voneinander ausgewählt unter Alkyl, Cycloalkyl, Aryl und Hetaryl, wie eingangs definiert.

Bevorzugt steht wenigstens einer der Reste R¹ und R² und besonders bevorzugt stehen R¹ und R² beide für Aryl, insbesondere beide für Phenyl.

Des Weiteren bevorzugt steht wenigstens einer der Reste R¹ und R² für eine über das pyrrolische Stickstoffatom an das Phosphoratom gebundene Pyrrolgruppe. Bevorzugt stehen R¹ und R² beide für eine über das pyrrolische Stickstoffatom an das Phosphoratom gebundene Pyrrolgruppe, wobei R¹ und R² für gleiche oder verschiedene Pyrrolgruppen stehen können.

Der Ausdruck "Pyrrolgruppe" steht im Sinne der vorliegenden Erfindung für eine Reihe unsubstituierter oder substituierter, heterocycloaromatischer Gruppen, die strukturell vom Pyrrolgrundgerüst abgeleitet sind und ein pyrrolisches Stickstoffatom im Heterocyclus enthalten, das kovalent mit einem Phosphoratom verknüpft werden kann. Der Ausdruck "Pyrrolgruppe" umfasst somit die unsubstituierten oder substituierten Gruppen Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, die im Falle einer Substitution im Allgemeinen 1, 2 oder 3, vorzugsweise 1 oder 2, besonders bevorzugt einen Substituenten, ausgewählt aus den Gruppen Alkyl, Alkoxy, Acyl, Carboxylat, Sulfonat, NE⁴E⁵, Alkylen-NE⁴E⁵ oder Halogen, tragen können. Bevorzugte Pyrrolgruppen sind 3-(C₁-C₄-Alkyl)indolylgruppen, wie die 3-Methylindolylgruppe (Skatolylgruppe).

Nach einer weiteren bevorzugten Ausführungsform sind R¹ und R² miteinander verbrückt. Dann steht die phosphoratomhaltige Gruppe vorzugsweise für eine Gruppe der Formel 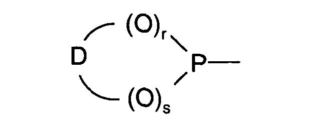 worin
- r: und s unabhängig voneinander für 0 oder 1 stehen, und
- D: zusammen mit dem Phosphoratom und dem/den Sauerstoffatom(en), an die es gebunden ist, für einen 5- bis 8-gliedrigen Heterocyclus steht, der gegebenenfalls ein-, zwei- oder dreifach mit Cycloalkyl, Heterocycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Halogen, Sulfonat, NE⁴E⁵, Alkylen-NE⁴E⁵, Nitro, Cyano und Carboxylat, tragen können und/oder D einen, zwei oder drei Substituenten, die ausgewählt sind unter Alkyl, Alkoxy, gegebenenfalls substituiertem Cycloalkyl und gegebenenfalls substituiertem Aryl, aufweisen kann und/oder D durch 1, 2 oder 3 gegebenenfalls substituierte Heteroatome unterbrochen sein kann.

Der Rest D steht vorzugsweise für eine C₂- bis C₆-Alkylenbrücke, die 1- oder 2-fach mit Aryl anelliert ist und/oder die einen Substituenten, der ausgewählt ist unter Alkyl, gegebenenfalls substituiertem Cycloalkyl und gegebenenfalls substituiertem Aryl, aufweisen kann und/oder die durch ein gegebenenfalls substituiertes Heteroatom unterbrochen sein kann.

Bei den anellierten Arylen der Reste D handelt es sich bevorzugt um Benzol oder Naphthalin. Anellierte Benzolringe sind vorzugsweise unsubstituiert oder weisen 1, 2 oder 3, insbesondere 1 oder 2 Substituenten auf, die vorzugsweise ausgewählt sind unter Alkyl, Alkoxy, Halogen, Sulfonat, NE⁴E⁵, Alkylen-NE⁴E⁵, Trifluormethyl, Nitro, Carboxylat, Alkoxycarbonyl, Acyl und Cyano. Anellierte Naphthaline sind vorzugsweise unsubstituiert oder weisen im nicht anellierten Ring und/oder im anellierten Ring jeweils 1, 2 oder 3, insbesondere 1 oder 2 der zuvor bei den anellierten Benzolringen genannten Substituenten auf. Bei den Substituenten der anellierten Aryle steht Alkyl vorzugsweise für C₁- bis C₄-Alkyl und insbesondere für Methyl, Isopropyl und tert.-Butyl. Alkoxy steht dabei vorzugsweise für C₁- bis C₄-Alkoxy und insbesondere für Methoxy. Alkoxycarbonyl steht vorzugsweise für C₁- bis C₄-Alkoxycarbonyl.

Wenn die C₂- bis C₆-Alkylenbrücke des Restes D durch 1, 2 oder 3, gegebenenfalls substituierte Heteroatome unterbrochen ist, so sind diese vorzugsweise ausgewählt unter O, S oder NR^{h}, wobei R^{h} für Alkyl, Cycloalkyl oder Aryl steht.

Wenn die C₂- bis C₆-Alkylenbrücke des Restes D substituiert ist, so weist sie vorzugsweise 1, 2 oder 3, insbesondere einen Substituenten auf, der/die ausgewählt ist/sind unter Alkyl, Cycloalkyl, Heterocycloalkyl, Aryl und Hetaryl, wobei die Cycloalkyl-, Heterocycloalkyl,-, Aryl- und Hetarylsubstituenten jeweils 1, 2 oder 3 der eingangs für diese Reste als geeignet genannten Substituenten tragen können.

Vorzugsweise steht der Rest D für eine C₃- bis C₆-Alkylenbrücke, die wie zuvor beschrieben anelliert und/oder substituiert und/oder durch gegebenenfalls substituierte Heteroatome unterbrochen ist. Insbesondere steht der Rest D für eine C₃- bis C₆-Alkylenbrücke, die ein- oder zweifach mit Phenyl und/oder Naphthyl anelliert ist, wobei die Phenyl- oder Naphthylgruppen 1, 2 oder 3 der zuvor genannten Substituenten tragen können.

Vorzugsweise steht der Rest D zusammen mit dem Phosphoratom und dem/den Sauerstoffatom(en), an die er gebunden ist, für einen 5- bis 8-gliedrigen Heterocyclus, wobei D für einen Rest steht, der ausgewählt ist unter den Resten der Formeln II.1 bis II.5, 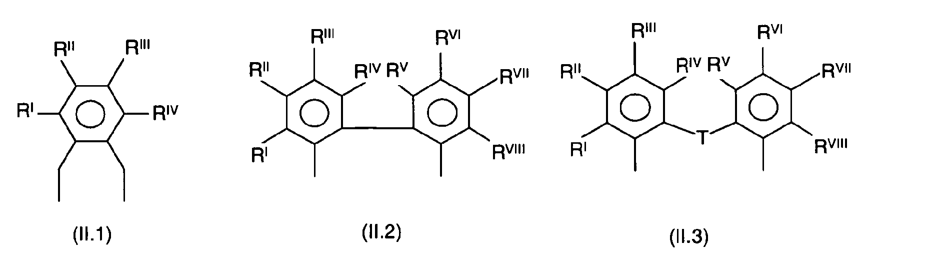 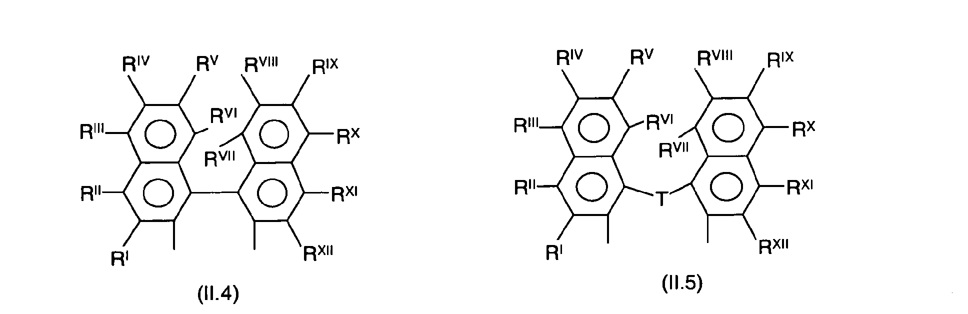 worin
- T: für O, S oder NRⁱ steht, wobei
Rⁱ für Alkyl, Cycloalkyl oder Aryl steht,
- oder T: für eine C₁- bis C₃-Alkylenbrücke steht, die eine Doppelbindung und/oder einen Alkyl-, Cycloalkyl- oder Arylsubstituenten aufweisen kann, wobei der Arylsubstituent einen, zwei oder drei der für Aryl genannten Substituenten tragen kann,
- oder T: für eine C₂- bis C₃-Alkylenbrücke steht, die durch O, S oder NRⁱ unterbrochen ist,
R^{I}, R^{II}, R^{III}, R^{IV}, R^{V}, R^{VI}, R^{VII}, R^{VIII}, R^{IX}, R^{X}, R^{XI} und R^{XII} unabhängig voneinander für Wasserstoff, Alkyl, Cycloalkyl, Aryl, Alkoxy, Halogen, Sulfonat, NE⁴E⁵, Alkylen-NE⁴E⁵, Trifluormethyl, Nitro, Alkoxycarbonyl oder Cyano stehen.

Bevorzugt weist wenigstens einer der erfindungsgemäß eingesetzten Liganden eine zur Tautomerie und zur Ausbildung intermolekularer nichtkovalenter Bindungen befähigte funktionelle Gruppe auf. Diese ist vorzugsweise ausgewählt unter Gruppen der Formel 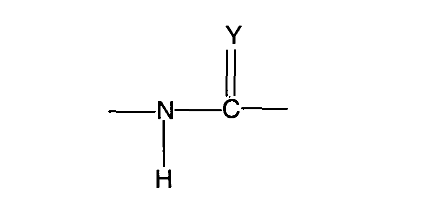 und den Tautomeren davon, worin Y für O, S oder NR⁴ steht, wobei R⁴ für Wasserstoff, Alkyl, Cycloalkyl, Heterocycloalkyl, Aryl oder Hetaryl steht.

Die Lage des jeweiligen Tautomeriegleichgewichts ist unter anderem abhängig von der Gruppe Y sowie den Substituenten an der zur Tautomerie befähigten Gruppe. Sie werden im Folgenden beispielhaft für die Keto-Enol-Tautomerie (speziell die Carbonsäureamid-Imidocarbonsäure-Tautomerie und die Amidin-Tautomerie) dargestellt: 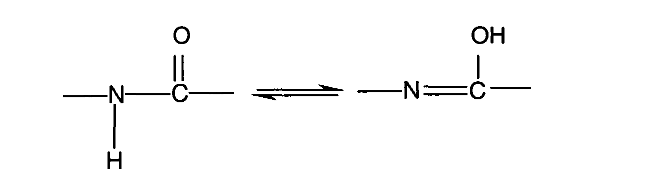 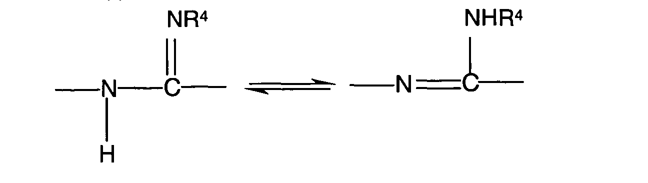 Vorzugsweise weisen die erfindungsgemäß eingesetzten Liganden wenigstens ein Strukturelement der allgemeinen Formeln I.a oder I.b 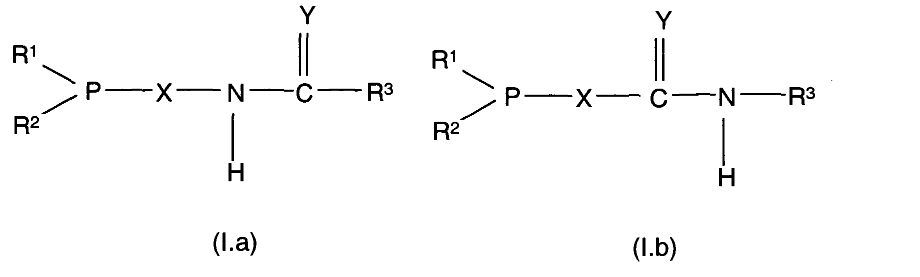 oder Tautomeren davon auf, worin
- R¹ und R²: unabhängig voneinander für Alkyl, Alkoxy, Cycloalkyl, Cycloalkoxy, Heterocycloalkyl, Heterocycloalkoxy, Aryl, Aryloxy, Hetaryl oder Hetaryloxy stehen,
- R³: für Wasserstoff steht oder eine der für R¹ und R² angegebenen Bedeutungen besitzt,
- X: für eine zweiwertige verbrückende Gruppe mit 1 bis 5 Brückenatomen zwischen den flankierenden Bindungen steht,
- Y: für O oder NR⁴ steht, wobei R⁴ für Wasserstoff, Alkyl, Cycloalkyl, Heterocycloalkyl, Aryl oder Hetaryl steht,
wobei zwei oder mehr als zwei der Reste X und R¹ bis R⁴ gemeinsam mit dem Strukturelement der Formel I.a oder I.b, an das sie gebunden sind, für eine mono- oder polycyclische Verbindung stehen können.

Bezüglich geeigneter und bevorzugter Reste R¹ und R² wird auf die vorherigen Ausführungen Bezug genommen.

Bevorzugt weist die zweiwertige verbrückende Gruppe X 1 bis 4, besonders bevorzugt 1 bis 3 Brückenatome zwischen den flankierenden Bindungen auf.

Bevorzugt steht die zweiwertige verbrückende Gruppe X für eine C₁-C₅-Alkylen-Brücke, die, abhängig von der Anzahl der Brückenatome, eine oder zwei Doppelbindungen und/oder einen, zwei, drei oder vier Substituenten, die ausgewählt sind unter Alkyl, Cycloalkyl, Heterocycloalkyl, Aryl, Hetaryl, Carboxylat, Sulfonat, Phosphonat, NE¹E² (E¹, E² = Wasserstoff, Alkyl, Cycloalkyl, Acyl oder Aryl), Hydroxy, Thiol, Halogen, Nitro, Acyl oder Cyano, aufweisen kann, wobei die Cycloalkyl-, Aryl- und Hetaryl-Substituenten zusätzlichen einen, zwei oder drei Substituenten, die ausgewählt sind unter Alkyl, Alkoxy, Halogen, Trifluormethyl, Nitro, Alkoxycarbonyl oder Cyano, tragen können und/oder ein oder zwei nichtbenachbarte Brückenatome der C₁-C₅-Alkylen-Brücke X durch ein Heteroatom oder eine Heteroatom-haltige Gruppe ersetzt sein können und/oder die Alkylen-Brücke X ein- oder zweifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E² (E¹ und E² = Wasserstoff, Alkyl, Cycloalkyl, Acyl oder Aryl) tragen können und/oder zwei oder mehr als zwei Brückenatome der C₁-C₅-Alkylen-Brücke X gemeinsam mit dem Strukturelement der Formel I.a oder I.b, an das sie gebunden sind, für eine mono- oder polycyclische Verbindung stehen können.

Bevorzugt steht X für eine C₁-C₅-Alkylen-Brücke, die eine oder zwei Doppelbindungen aufweisen kann. Des Weiteren bevorzugt können zwei oder mehr als zwei der Brückenatome der Brücke X gemeinsam mit dem Strukturelement der Formel I.a oder I.b, an das sie gebunden sind, für eine mono- oder polycyclische Verbindung stehen.

Vorzugsweise weisen die erfindungsgemäß eingesetzten Liganden wenigstens ein Strukturelement der allgemeinen Formeln I.a oder I.b auf, worin die Gruppe X und der Rest R³ gemeinsam mit der Gruppe -NH-C(=Y)-, an die sie gebunden sind, für einen 5- bis 8-gliedrigen, vorzugsweise 6-gliedrigen Ring stehen. Dieser Ring kann eine, zwei oder drei Doppelbindungen aufweisen, wobei eine dieser Doppelbindungen auf der tautomeren Gruppe -N=C(YH)- beruhen kann. Bevorzugt sind 6-gliedrige Ringe, die unter Berücksichtigung der Tautomerie drei Doppelbindungen aufweisen. Derartige Ringsysteme, bei denen eines der Tautomeren ein aromatisches Ringsystem ausbilden kann, sind besonders stabil. Die genannten Ringe können unsubstituiert sein oder einen, zwei, drei, vier oder fünf der zuvor genannten Substituenten aufweisen. Diese sind vorzugsweise ausgewählt unter C₁-C₄-Alkyl, besonders bevorzugt Methyl, Ethyl, Isopropyl oder tert.-Butyl, C₁-C₄-Alkoxy, speziell Methoxy, Ethoxy, Isopropyloxy oder tert.-Butyloxy, sowie Aryl, vorzugsweise Phenyl. In einer geeigneten Ausführungsform weisen die genannten Ringe wenigstens eine Doppelbindung auf, wobei die an diese Doppelbindung gebundenen Reste für ein kondensiertes Ringsystem mit 1, 2 oder 3 weiteren Ringen steht. Dabei handelt es sich bevorzugt um Benzol oder Naphthalinringe. Anellierte Benzolringe sind vorzugsweise unsubstituiert oder weisen 1, 2 oder 3 Substituenten auf, die ausgewählt sind unter Alkyl, Alkoxy, Carboxylat, Sulfonat, Halogen, NE¹E², Trifluormethyl, Nitro, Alkoxycarbonyl, Acyl und Cyano. Anellierte Naphthalinringe sind vorzugsweise unsubstituiert oder weisen im nichtanellierten und/oder im anellierten Ring je 1, 2 oder 3 der zuvor bei den anellierten Benzolringen genannten Substituenten auf.

Vorzugsweise sind die erfindungsgemäß eingesetzten Liganden ausgewählt unter Verbindungen der allgemeinen Formeln I.1 bis I.3 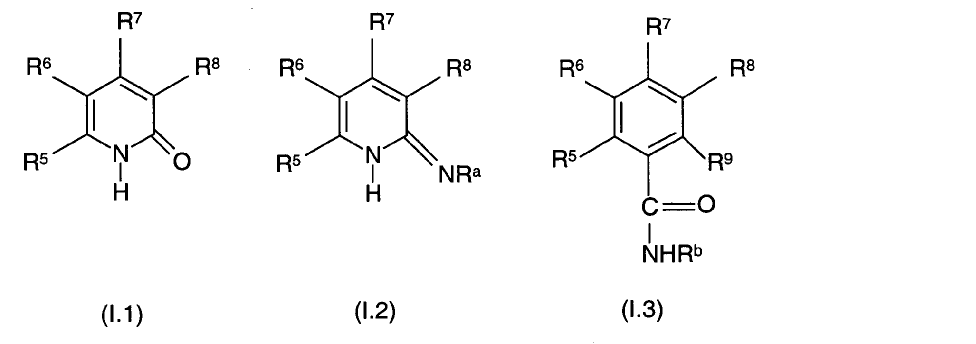 und den Tautomeren davon, worin
einer der Reste R⁵ bis R⁹ für eine Gruppe der Formel

―W'―PR¹R²

steht, worin
- W': für eine Einfachbindung, ein Heteroatom, eine Heteroatom-haltige Gruppe oder eine zweiwertige verbrückende Gruppe mit 1 bis 4 Brückenatomen zwischen den flankierenden Bindungen steht,
- R¹ und R²: wie zuvor definiert sind,
die Reste R⁵ bis R⁹, die nicht für -W'-PR¹R² stehen, unabhängig voneinander für Wasserstoff, Alkyl, Cycloalkyl, Heterocycloalkyl, Aryl, Hetaryl, WCOOR^{°}, WCOO⁻ M⁺, W(SO₃)R^{°} W(SO₃)⁻M⁺, WPO₃(R^{°})(R^{p}), W(PO₃)²⁻(M⁺)₂, WNE'E², W(NE¹E²E³)⁺X⁻, WOR^{q}, WSR^{q}, (CHR^{r}CH₂O)ₓR^{q}, (CH₂NE¹)ₓR^{q}, (CH₂CH₂NE¹)ₓR^{q}, Halogen, Nitro, Acyl oder Cyano stehen,
worin
- W: für eine Einfachbindung, ein Heteroatom, eine Heteroatom-haltige Gruppe oder eine zweiwertige verbrückende Gruppe mit 1 bis 20 Brückenatomen steht,
- R^{°} und R^{p}: jeweils gleiche oder verschiedene Reste, ausgewählt unter Alkyl, Cycloalkyl, Acyl oder Aryl bedeuten,
- R^{q}, E¹, E², E³: jeweils gleiche oder verschiedene Reste, ausgewählt unter Wasserstoff, Alkyl, Cycloalkyl, Acyl oder Aryl bedeuten,
- R^{r}: für Wasserstoff, Methyl oder Ethyl steht,
- M⁺: für ein Kationäquivalent steht,
- X⁻: für ein Anionäquivalent steht und
- x: für eine ganze Zahl von 1 bis 240 steht,
wobei jeweils zwei benachbarte Reste R⁵, R⁶, R⁷, R⁸ und R⁹ zusammen mit den Ringkohlenstoffatomen, an die sie gebunden sind, auch für ein kondensiertes Ringsystem mit 1, 2 oder 3 weiteren Ringen stehen können, und
R^{a} und R^{b} für Wasserstoff, Alkyl, Acyl, Cycloalkyl, Heterocycloalkyl, Aryl oder Hetaryl stehen.

In einer geeigneten Ausführungsform des erfindungsgemäßen Verfahrens werden Liganden der allgemeinen Formeln I.1 bis I.3 eingesetzt, worin R⁵ und R⁶ und/oder R⁷ und R⁸ für ein kondensiertes Ringsystem mit 1, 2 oder 3 weiteren Ringen stehen. Wenn R⁵ und R⁶ und/oder R⁷ und R⁸ für ein ankondensiertes, also anelliertes Ringsystem stehen, so handelt es sich bevorzugt um Benzol- oder Naphthalinringe. Anellierte Benzolringe sind vorzugsweise unsubstituiert und weisen 1, 2 oder 3, insbesondere 1 oder 2 Substituenten auf, die vorzugsweise ausgewählt sind unter Alkyl, Alkoxy, Halogen, SO₃H, Sulfonat, NE¹E², Alkylen-NE¹E², Trifluormethyl, Nitro, COOR^{°}, Alkoxycarbonyl, Acyl und Cyano. Anellierte Naphthalinringe sind vorzugsweise unsubstituiert oder weisen im nichtanellierten Ring und/oder im anellierten Ring je 1, 2 oder 3, insbesondere 1 oder 2 der zuvor bei den anellierten Benzolringen genannten Substituenten auf. Bevorzugt stehen R⁷ und R⁸ für ein ankondensiertes Ringsystem. Dann stehen R⁶ und R⁹ vorzugsweise beide für Wasserstoff oder steht einer dieser Reste für Wasserstoff und der andere für einen Substituenten, der ausgewählt ist unter C₁- bis C₈-Alkyl, vorzugsweise C₁- bis C₄-Alkyl, speziell Methyl, Ethyl, Isopropyl oder tert.-Butyl.

In den Verbindungen der Formeln 1.1 bis 1.3 steht vorzugsweise der Rest R⁵ für eine Gruppe der Formel -W'-PR¹R², wie zuvor definiert.

Vorzugsweise steht in den Gruppen der Formel -W'-PR¹R² W' für ein Sauerstoffatom oder eine Einfachbindung zwischen der Gruppe PR¹R² und einem Ringkohlenstoffatom, an das diese Gruppe gebunden ist.

Bevorzugt stehen in den Verbindungen der Formeln 1.1 bis 1.3 die Reste R¹ und R² unabhängig voneinander für C₁-C₈-Alkyl, wie Methyl, Ethyl, Isopropyl und tert.-Butyl, C₅-C₈-Cycloalkyl, wie Cyclohexyl, oder Aryl, wie Phenyl. Bevorzugt stehen die Reste R¹ und R² beide für Aryl, insbesondere beide für Phenyl.

Vorzugsweise sind in den Verbindungen 1.1 bis 1.3 die Reste R⁶, R⁷, R⁸ und R⁹ unabhängig voneinander ausgewählt unter Wasserstoff, C₁-C₄-Alkyl, C₁-C₄-Alkoxy, Carboxylat, Sulfonat, NE¹E², Halogen, Trifluormethyl, Nitro, Alkoxycarbonyl, Acyl und Cyano.

Bevorzugt stehen R⁶, R⁷, R⁸ und R⁹ für Wasserstoff. Des Weiteren bevorzugt stehen die Reste R⁷ und R⁸ zusammen mit den Ringkohlenstoffatomen, an die sie gebunden sind, für ein ankondensiertes Ringsystem, wie zuvor definiert, insbesondere für einen Benzolring. Dann stehen die Reste R⁶ und, falls vorhanden, R⁹ vorzugsweise für Wasserstoff.

Bevorzugt steht in der Verbindung der Formel 1.2 der Rest R^{a} für Wasserstoff, C₁-C₈-Alkyl, wie Methyl, Ethyl, Isopropyl und tert.-Butyl, Acyl, C₅-C₈-Cycloalkyl, wie Cyclohexyl, oder C₆-C₁₀-Aryl, wie Phenyl. Besonders bevorzugt steht R^{a} für Acyl, insbesondere für Alkanoyl, wie Acetyl, Propanoyl, Butanoyl, Isobutanoyl und Pivaloyl.

Bevorzugt steht in den Verbindungen der Formel 1.3 der Rest R^{b} für Wasserstoff, C₁-C₈-Alkyl, wie Methyl, Ethyl, Isopropyl und tert.-Butyl, C₅-C₈-Cycloalkyl, wie Cyclohexyl, C₆-C₁₀-Aryl, wie Phenyl, oder Hetaryl.

Die Verbindungen der Formeln 1.1 bis 1.3 (wie zuvor definiert und im Folgenden genauer ausgeführt) eignen sich auch unabhängig von ihrer Befähigung zur Ausbildung intermolekularer, nichtkovalenter Bindungen als Liganden in Hydroformylierungskatalysatoren. Gegenstand der Erfindung ist daher auch ein Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Katalysators, umfassend wenigstens einen Komplex eines Metalls der VIII. Nebengruppe des Periodensystems der Elemente mit Liganden, die ausgewählt sind unter Verbindungen der allgemeinen Formeln I.1 bis I.3.

Vorzugsweise sind die erfindungsgemäß eingesetzten Liganden ausgewählt unter Verbindungen der allgemeinen Formeln I.i bis I.iii 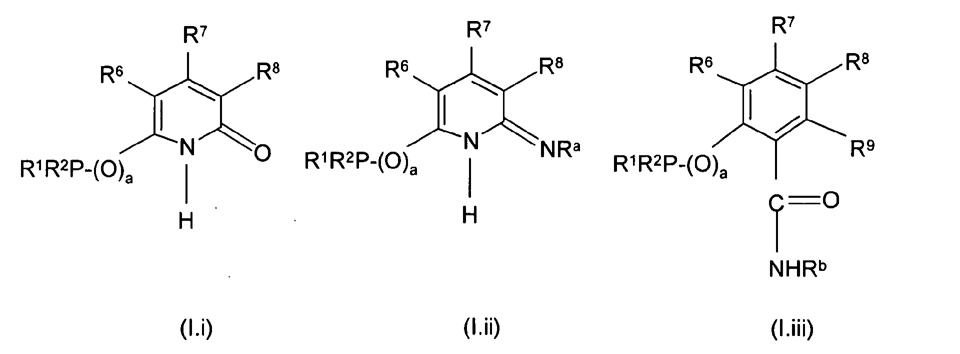 und den Tautomeren davon, worin
a für 0 oder 1 steht,
- R¹ und R²: wie zuvor definiert sind,
- R⁶ bis R⁹: unabhängig voneinander für Wasserstoff, C₁-C₄-Alkyl, C₁-C₄-Alkoxy, Acyl, Aryl, Heteroaryl, Halogen, C₁-C₄-Alkoxycarbonyl oder Carboxylat stehen,
wobei jeweils zwei benachbarte Reste R⁶, R⁷, R⁸ und R⁹ zusammen mit den Ringkohlenstoffatomen, an die sie gebunden sind, auch für ein kondensiertes Ringsystem mit 1, 2 oder 3 weiteren Ringen stehen können, und
- R^{a} und R^{b}: für Wasserstoff, Alkyl, Acyl, Cycloalkyl oder Aryl stehen.

Bevorzugt stehen in den Verbindungen der Formeln I.i bis I.iii die Reste R¹ und R² unabhängig voneinander für C₁-C₈-Alkyl, wie Methyl, Ethyl, Isopropyl und tert.-Butyl, C₅-C₈-Cycloalkyl, wie Cyclohexyl, oder Aryl, wie Phenyl. Bevorzugt stehen die Reste R¹ und R² beide für Aryl, insbesondere beide für Phenyl.

Vorzugsweise sind in den Verbindungen I.i bis I.iii die Reste R⁶, R⁷, R⁸ und R⁹ unabhängig voneinander ausgewählt unter Wasserstoff, C₁-C₄-Alkyl, C₁-C₄-Alkoxy, Carboxylat, Sulfonat, NE¹E², Halogen, Trifluormethyl, Nitro, Alkoxycarbonyl, Acyl und Cyano. Bevorzugt stehen R⁶, R⁷, R⁸ und R⁹ für Wasserstoff. Des Weiteren bevorzugt stehen die Reste R⁷ und R⁸ zusammen mit den Ringkohlenstoffatomen, an die sie gebunden sind, für ein ankondensiertes Ringsystem, wie zuvor definiert, insbesondere für einen Benzolring. Dann stehen R⁶ und, falls vorhanden, R⁹ vorzugsweise für Wasserstoff.

Bevorzugt steht in der Verbindung der Formel I.ii der Rest R^{a} für Wasserstoff, C₁-C₈-Alkyl, wie Methyl, Ethyl, Isopropyl und tert.-Butyl, C₅-C₈-Cycloalkyl, wie Cyclohexyl, oder C₆-C₁₀-Aryl, wie Phenyl. Besonders bevorzugt steht R^{a} für Acyl, insbesondere für Alkanoyl, wie Acetyl, Propanoyl, Butanoyl, Isobutanoyl und Pivaloyl.

Bevorzugt steht in den Verbindungen der Formel I.iii der Rest R^{b} für Wasserstoff, C₁-C₈-Alkyl, wie Methyl, Ethyl, Isopropyl und tert.-Butyl, C₅-C₈-Cycloalkyl, wie Cyclohexyl, C₆-C₁₀-Aryl, wie Phenyl, oder Hetaryl.

Im Folgenden werden einige vorteilhafte Verbindungen aufgelistet, wozu auch deren Tautomere zählen:

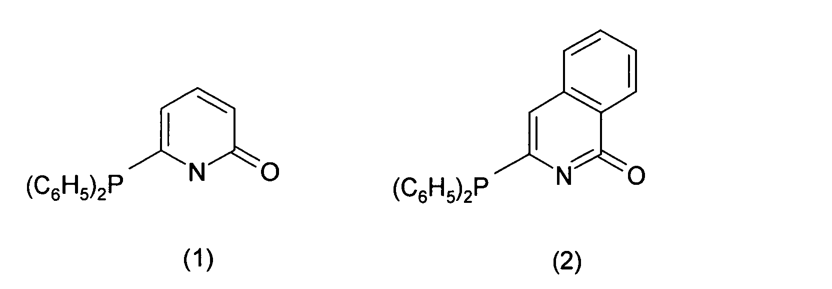

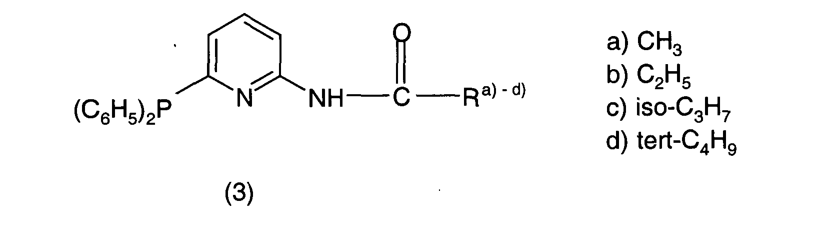

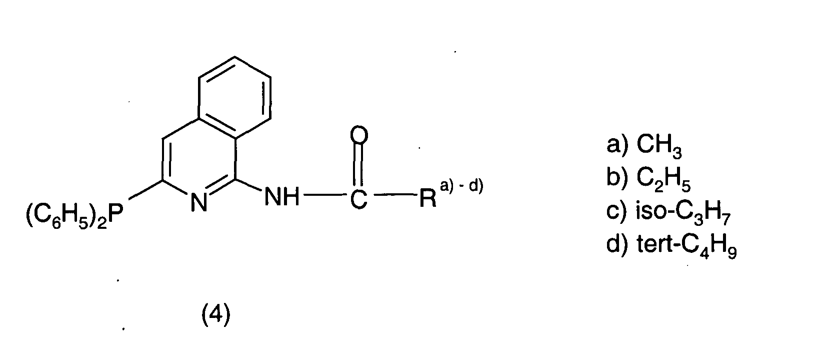

Beispielhaft für einen erfindungsgemäß besonders vorteilhaft einsetzbaren Liganden sei 6-Diphenylphosphino-1-H-pyridin-2-on genannt.

Die zuvor genannten Liganden der Formeln 1.1 bis 1.3, speziell der Formeln I.i bis I.iii und spezieller der Formeln (1) bis (4) eignen sich sowohl als alleinige Liganden, wobei eine Homo-Dimerbildung angenommen wird, als auch in Liganden-Kombinationen, wobei eine zumindest teilweise Hetero-Dimerbildung angenommen wird. Bei Liganden-Kombinationen können alle Liganden ausgewählt sein unter Liganden der Formeln I.1 bis I.3 und speziell unter Liganden der Formeln I.i bis I.iii, spezieller (1) bis (4). Es ist jedoch auch möglich, nur wenigstens einen der Liganden einer Liganden-Kombination unter Liganden der genannten Formeln auszuwählen und mit wenigstens einem davon verschiedenen Liganden zu kombinieren. Zur Kombination (als eine Komponente eines Hetero-Dimers) eignen sich vorzugsweise Liganden, die ausgewählt sind unter Verbindungen der folgenden Formel II 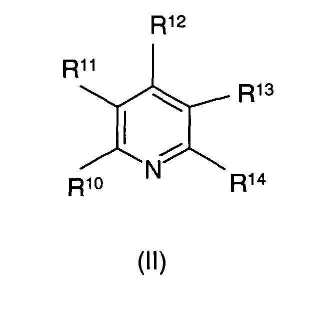 worin
einer der Reste R¹⁰ bis R¹⁴ für eine Gruppe der Formel

―W"―PR¹R²

steht, worin
- W": für eine Einfachbindung, ein Heteroatom, eine Heteroatom-haltige Gruppe oder eine zweiwertige verbrückende Gruppe mit 1 bis 4 Brückenatomen zwischen den flankierenden Bindungen steht,
- R¹ und R²: unabhängig voneinander für Alkyl, Alkoxy, Cycloalkyl, Cycloalkoxy, Heterocycloalky, Heterocycloalkoxy, Aryl, Aryloxy, Hetaryl oder Hetaryloxy stehen,
die Reste R¹⁰ bis R¹⁴, die nicht für ―W"―PR¹R² stehen, unabhängig voneinander für Wasserstoff, Alkyl, Cycloalkyl, Heterocycloalkyl, Aryl, Hetaryl, WCOOR^{s}, WCOO⁻ M⁺, W(SO₃)R^{s}, W(SO₃)⁻M⁺, WPO₃(R^{s})(R^{t}), W(PO₃)²⁻(M⁺)₂, WNE⁷E⁸, W(NE⁷E⁸E⁹)⁺X⁻, WOR^{u}, WSR^{u}, (CHR^{v}CH₂O)₂R^{u}, (CH₂NE⁷)_{z}R^{u}, (CH₂CH₂NE⁷)_{z}R^{u}, Halogen, Nitro, Acyl oder Cyano stehen,
worin
- W: für eine Einfachbindung, ein Heteroatom, eine Heteroatom-haltige Gruppe oder eine zweiwertige verbrückende Gruppe mit 1 bis 20 Brückenatomen steht,
- R^{s} und R^{t}: jeweils gleiche oder verschiedene Reste, ausgewählt unter Alkyl, Cycloalkyl, Acyl oder Aryl bedeuten,
- R^{u}, E⁷, E⁸, E⁹: jeweils gleiche oder verschiedene Reste, ausgewählt unter Wasserstoff, Alkyl, Cycloalkyl, Acyl oder Aryl bedeuten,
- R^{v}: für Wasserstoff, Methyl oder Ethyl steht,
- M⁺: für ein Kationäquivalent steht,
- X⁻: für ein Anionäquivalent steht und
- z: für eine ganze Zahl von 1 bis 240 steht,
wobei jeweils zwei benachbarte Reste R¹⁰, R¹¹, R¹², R¹³ und R¹⁴ zusammen mit den Ringkohlenstoffatomen, an die sie gebunden sind, auch für ein kondensiertes Ringsystem mit 1, 2 oder 3 weiteren Ringen stehen können.

In den Verbindungen der Formel II steht vorzugsweise der Rest R¹⁰ für eine Gruppe der Formel -W"-PR¹R², wie zuvor definiert.

Vorzugsweise steht in den Gruppen der Formel -W"-PR¹R² W" für ein Sauerstoffatom oder eine Einfachbindung zwischen der Gruppe PR¹R² und einem Ringkohlenstoffatom, an das diese Gruppe gebunden ist.

Bevorzugt stehen in den Verbindungen der Formel II die Reste R¹und R² unabhängig voneinander für C₁-C₈-Alkyl, wie Methyl, Ethyl, Isopropyl und tert.-Butyl, C₅-C₈-Cycloalkyl, wie Cyclohexyl, oder Aryl, wie Phenyl. Bevorzugt stehen die Reste R¹ und R² beide für Aryl, insbesondere beide für Phenyl.

Vorzugsweise sind in den Verbindungen der Formel II die Reste R¹¹, R¹², R¹³ und R¹⁴ unabhängig voneinander ausgewählt unter Wasserstoff, C₁-C₄-Alkyl, C₁-C₄-Alkoxy, Carboxylat, Sulfonat, NE¹E², Halogen, Trifluormethyl, Nitro, Alkoxycarbonyl, Acyl und Cyano. Bevorzugt stehen R⁶, R⁷, R⁸ und R⁹ für Wasserstoff. Des Weiteren bevorzugt stehen die Reste R¹² und R¹³ zusammen mit den Ringkohlenstoffatomen, an die sie gebunden sind, für ein ankondensiertes Ringsystem, wie zuvor definiert, insbesondere für einen Benzolring. Dann stehen die Reste R¹¹ und R¹⁴ vorzugsweise für Wasserstoff. Eine bevorzugte Verbindung der Formel II ist 2-(Diphenylphosphino)-pyridin.

Zur Veranschaulichung werden im Folgenden einige bevorzugte Ligand/Ligand-Paare aufgeführt:

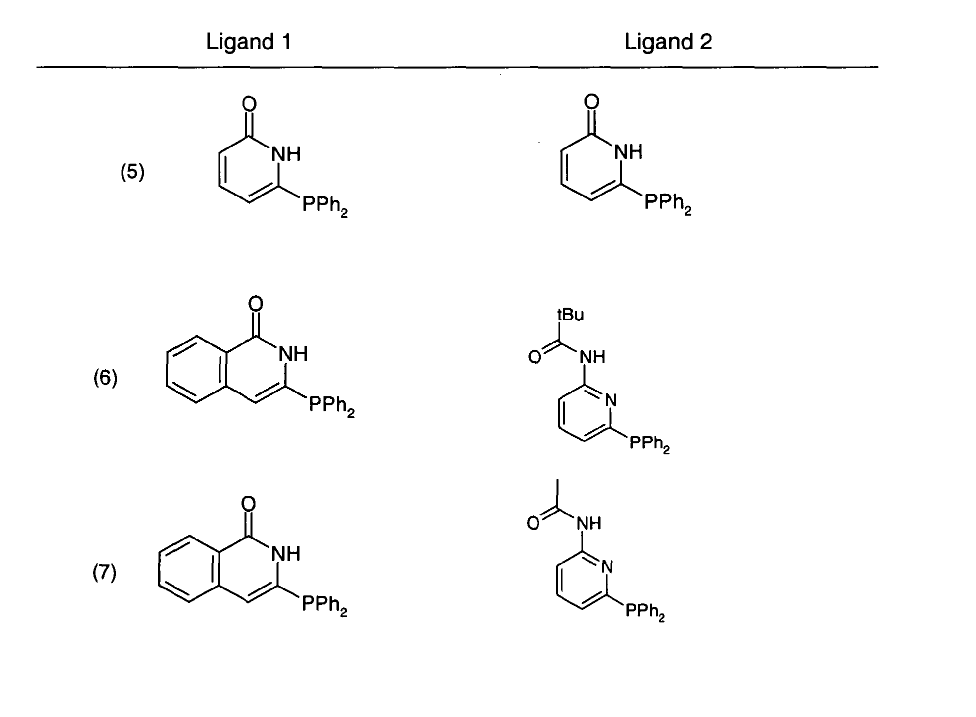

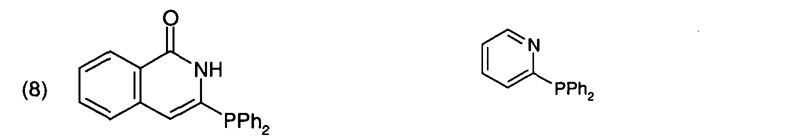

Die Herstellung von erfindungsgemäß einsetzbaren Liganden kann nach üblichen, dem Fachmann bekannten Verfahren erfolgen.

M. Akazome et al. beschreiben in J. Org. Chem. 2000, 65, S. 6917-6921 die Synthese von (2-Oxo-1,2-dihydro-x-pyridyl)diphenylphosphinen mit x = 3, 5 und 6 (3-, 5- und 6-Diphenylphosphino-2-pyridinonen) durch Lithiierung der entsprechenden 2-Benzoyloxy-x-brompyridine, nachfolgende Kupplung mit Chlordiphenylphosphin und abschließende Abspaltung der Benzoyl-Schutzgruppe mit Trifluoressigsäure. Bezüglich der Herstellung der als Edukte eingesetzten 2-Benzoyloxy-x-brompyridine wird auf das von Y. Dycharme und J. D. Wüst in J. Org. Chem. 1988, 53, S. 5787 beschriebene Verfahren Bezug genommen.

G. R. Newkome und D. C. Hager beschreiben in J. Org. Chem. 1978, 43, S. 947-949 ein Verfahren zur Herstellung von Pyridyldiphenylphosphinen durch Umsetzung von Lithiumdiphenylphosphit mit Halogenpyridinen. Danach wird 6-Diphenylphosphinopyridinon aus Lithiumdiphenylphosphid und 6-Chlor-2-methoxypyridin erhalten.

Auf die zuvor genannten Dokumente wird in vollem Umfang Bezug genommen.

Ein neues Verfahren zur Herstellung von Phosphinopyridinonen und/oder Tautomeren davon umfasst, dass man eine Pyridinverbindung, die eine geschützte Hydroxylgruppe und eine nucleophil verdrängbare Gruppe trägt, mit einer Lösung eines Phosphins und eines Alkalimetalls in flüssigem Ammoniak unter Erhalt wenigstens einer Pyridinverbindung, die eine geschützte Hydroxylgruppe und eine Phosphinogruppe trägt, umsetzt und anschließend die Schutzgruppe der Hydroxylgruppe abspaltet. Dieses Verfahren ist nicht Gegenstand der vorliegenden Anmeldung, sondern der parallelen deutschen Patentanmeldung 103 13 320.8, auf die Bezug genommen wird.

Ein weiterer Gegenstand der Erfindung ist ein Katalysator, umfassend wenigstens einen Komplex eines Metalls der VIII. Nebengruppe des Periodensystems der Elemente mit Liganden, die jeweils eine phosphoratomhaltige Gruppe und wenigstens eine zur Ausbildung intermolekularer nichtkovalenter Bindungen befähigte funktionelle Gruppe aufweisen, wobei der Komplex über intermolekulare nichtkovalente Bindungen dimerisierte Liganden aufweist und wobei der Abstand zwischen den Phosphoratomen der dimerisierten Liganden höchstens 5 Å beträgt. Auf die vorherigen Ausführungen zu geeigneten und bevorzugten Liganden wird vollständig Bezug genommen.

Ein weiterer Gegenstand der Erfindung ist ein Katalysator, umfassend wenigstens einen Komplex eines Metalls der VIII. Nebengruppe des Periodensystems mit wenigstens einem Liganden, der ausgewählt ist unter Verbindungen der allgemeinen Formeln I.1 bis I.3, wie zuvor definiert. Auf die vorherigen Ausführungen zu geeigneten und bevorzugten Liganden I.1 bis I.3 wird vollständig Bezug genommen.

Die erfindungsgemäßen und erfindungsgemäß eingesetzten Katalysatoren weisen vorzugsweise zwei oder mehr als zwei der zuvor beschriebenen Verbindungen als Liganden auf. Dabei liegen bevorzugt wenigstens zwei der Liganden in dimerisierter Form vor. Zusätzlich zu den zuvor beschriebenen Liganden können sie noch wenigstens einen weiteren Liganden, der vorzugsweise ausgewählt ist unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit- und Phosphitliganden aufweisen.

Bevorzugt handelt es sich bei dem Metall der VIII. Nebengruppe um Cobalt, Ruthenium, Rhodium, Palladium, Platin, Osmium oder Iridium und insbesondere um Cobalt, Rhodium, Ruthenium und Iridium.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für eine phosphorhaltige Verbindung der Formel I und q, x, y, z für ganze Zahlen, abhängig von der Wertigkeit und Art des Metalls sowie der Bindigkeit des Liganden L, stehen. Vorzugsweise stehen z und q unabhängig voneinander mindestens für einen Wert von 1, wie z. B. 1, 2 oder 3. Die Summe aus z und q steht bevorzugt für einen Wert von 1 bis 5. Dabei können die Komplexe gewünschtenfalls zusätzlich noch mindestens einen der zuvor beschriebenen weiteren Liganden aufweisen. Es besteht Grund zu der Annahme, dass auch die katalytisch aktive Spezies dimerisierte Liganden (Pseudochelate) aufweist.

Nach einer bevorzugten Ausführungsform werden die Hydroformylierungskatalysatoren in situ, in dem für die Hydroformylierungsreaktion eingesetzten Reaktor, hergestellt. Gewünschtenfalls können die erfindungsgemäßen Katalysatoren jedoch auch separat hergestellt und nach üblichen Verfahren isoliert werden. Zur in situ-Herstellung der erfindungsgemäßen Katalysatoren kann man z. B. wenigstens einen erfindungsgemäß eingesetzten Liganden, eine Verbindung oder einen Komplex eines Metalls der VIII. Nebengruppe, gegebenenfalls wenigstens einen weiteren zusätzlichen Liganden und gegebenenfalls ein Aktivierungsmittel in einem inerten Lösungsmittel unter den Hydroformylierungsbedingungen umsetzen.

Geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)-säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)-oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, im erfindungsgemäßen Verfahren verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltacetat, Cobaltethylhexanoat, Cobaltnaphthanoat, sowie der Cobalt-Caproat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weitere geeignete Verbindungen des Cobalts, Rhodiums, Rutheniums und Iridiums sind im Prinzip bekannt und in der Literatur hinreichend beschrieben oder sie können vom Fachmann analog zu den bereits bekannten Verbindungen hergestellt werden.

Geeignete Aktivierungsmittel sind z. B. Brönsted-Säuren, Lewis-Säuren, wie z. B. BF₃, AlCl₃, ZnCl₂, und Lewis-Basen.

Als Lösungsmittel werden vorzugsweise die Aldehyde eingesetzt, die bei der Hydroformylierung der jeweiligen Olefine entstehen, sowie deren höher siedende Folgereaktionsprodukte, z. B. die Produkte der Aldolkondensation. Ebenfalls geeignete Lösungsmittel sind Aromaten, wie Toluol und Xylole, Kohlenwasserstoffe oder Gemische von Kohlenwasserstoffen, auch zum Verdünnen der oben genannten Aldehyde und der Folgeprodukte der Aldehyde. Weitere Lösungsmittel sind Ester aliphatischer Carbonsäuren mit Alkanolen, beispielsweise Essigester oder Texanol^{TM}, Ether wie tert.-Butylmethylether und Tetrahydrofuran.

Weiterhin ist es möglich die Umsetzungen auch in Wasser oder wässrigen Lösungsmittelsystemen durchzuführen, die neben Wasser ein mit Wasser mischbares Lösungsmittel, beispielsweise ein Keton wie Aceton und Methylethylketon oder ein anderes Lösungsmittel enthalten. Zu diesem Zweck setzt man Liganden ein, die mit polaren Gruppen, beispielsweise ionischen Gruppen wie SO₃₋M⁺, CO₂₋M⁺ mit M⁺ = Na⁺, K⁺ oder NH₄⁺ oder wie N(CH₃)₄⁺modifiziert sind. Die Umsetzungen erfolgen dann im Sinne einer Zweiphasenkatalyse, wobei der Katalysator sich in der wässrigen Phase befindet und Einsatzstoffe und Produkte die organische Phase bilden. Auch die Umsetzung in den "lonic Liquids" kann als Zweiphasenkatalyse ausgestaltet sein.

Das Molmengenverhältnis von Monophosphorligand zu Metall der VIII. Nebengruppe liegt im Allgemeinen in einem Bereich von etwa 1:1 bis 1000:1, vorzugsweise 2:1 bis 500:1.

Als Substrate für das erfindungsgemäße Hydroformylierungsverfahren kommen prinzipiell alle Verbindungen in Betracht, welche eine oder mehrere ethylenisch ungesättigte Doppelbindungen enthalten. Dazu zählen z. B. Olefine, wie α-Olefine, interne geradkettige und interne verzweigte Olefine. Bevorzugt werden α-Olefine, wie z. B. Ethylen, Propen, 1-Buten, 1-Penten, 1-Hexen, 1-Hepten, 1-Octen, 1-Nonen, 1-Decen, 1-Undecen, 1-Dodecen etc. eingesetzt.

Geeignete verzweigte, interne Olefine sind vorzugsweise C₄-C₂₀-Olefine, wie 2-Methyl-2-buten, 2-Methyl-2-penten, 3-Methyl-2-penten, verzweigte, interne Hepten-Gemische, verzweigte, interne Octen-Gemische, verzweigte, interne Nonen-Gemische, verzweigte, interne Decen-Gemische, verzweigte, interne Undecen-Gemische, verzweigte, interne Dodecen-Gemische etc.

Geeignete zu hydroformylierende Olefine sind weiterhin C₅-C₈-Cycloalkene, wie Cyclopenten, Cyclohexen, Cyclohepten, Cycloocten und deren Derivate, wie z. B. deren C₁-C₂₀-Alkylderivate mit 1 bis 5 Alkylsubstituenten. Geeignete zu hydroformylierende Olefine sind weiterhin Vinylaromaten, wie Styrol, α-Methylstyrol, 4-lsobutylstyrol etc. Geeignete zu hydroformylierende Olefine sind weiterhin α,β-ethylenisch ungesättigte Mono- und/oder Dicarbonsäuren, deren Ester, Halbester und Amide, wie Acrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäuremethylester, Methacrylsäuremethylester, ungesättigte Nitrile, wie 3-Pentennitril, 4-Pentennitril, Acrylnitril, Vinylether, wie Vinylmethylether, Vinylethylether, Vinylpropylether etc., C₁-C₂₀-Alkenole, -Alkendiole und -Alkadienole, wie 2,7-Octadienol-1. Geeignete Substrate sind weiterhin Di- oder Polyene mit isolierten oder konjugierten Doppelbindungen. Dazu zählen z. B. 1,3-Butadien, 1,4-Pentadien, 1,5-Hexadien, 1,6-Heptadien, 1,7-Octadien, 1,8-Nonadien, 1,9-Decadien, 1,10-Undecadien, 1,11-Dodecadien, 1,12-Tridecadien, 1,13-Tetradecadien, Vinylcyclohexen, Dicyclopentadien, 1,5,9-Cyclooctatrien sowie Butadienhomo- und -copolymere.

In einer geeigneten Ausführung ist die zur Hydroformylierung eingesetzte ungesättigte Verbindung ausgewählt unter internen linearen Olefinen und Olefingemischen, die wenigstens ein internes lineares Olefin enthalten. Geeignete lineare (geradkettige) interne Olefine sind vorzugsweise C₄-C₂₀-Olefine, wie 2-Buten, 2-Penten, 2-Hexen, 3-Hexen, 2-Hepten, 3-Hepten, 2-Octen, 3-Octen, 4-Octen etc. und Mischungen davon.

Die erfindungsgemäßen und erfindungsgemäß eingesetzten Katalysatoren eignen sich auch in vorteilhafter Weise zur Hydroformylierung funktionalisierter Olefine, insbesondere funktionalisierter 1-Olefine. Bevorzugt sind die zu hydroformylierenden Olefine ausgewählt unter Verbindungen der allgemeinen Formel III

CH₂=CH―Z―(Fu)ₙ (III)

worin
- Z: für eine zweiwertige verbrückende Gruppe mit 1 bis 20 Brückenatomen zwischen den flankierenden Bindungen steht und
- Fu: für eine funktionelle Gruppe steht, und
- n: für eine ganze Zahl von 1 bis 4 steht.

Bevorzugt steht die zweiwertige verbrückende Gruppe Z für eine C₁-C₂₀-Alkylen-Brücke, die, abhängig von der Anzahl der Brückenatome, eine, zwei, drei oder vier Doppelbindungen und/oder einen, zwei, drei oder vier Substituenten aufweisen kann, wobei die Cycloalkyl-, Aryl- und Hetaryl-Substituenten ihrerseits zusätzlichen einen, zwei oder drei Substituenten, die ausgewählt sind unter Alkyl, Alkoxy, Halogen, Trifluormethyl, Nitro, Alkoxycarbonyl oder Cyano, tragen können und/oder 1 bis 10 nichtbenachbarte Brückenatome der C₁-C₂₀-Alkylen-Brücke Z durch ein Heteroatom oder eine Heteroatom-haltige Gruppe ersetzt sein können und/oder die Alkylen-Brücke Z einoder zweifach mit Aryl und/oder Hetaryl anelliert sein kann, wobei die anellierten Aryl- und Hetarylgruppen je einen, zwei oder drei Substituenten, die ausgewählt sind unter Alkyl, Cycloalkyl, Aryl, Alkoxy, Cycloalkoxy, Aryloxy, Acyl, Halogen, Trifluormethyl, Nitro, Cyano, Carboxyl, Alkoxycarbonyl oder NE¹E² (E¹ und E² = Wasserstoff, Alkyl, Cycloalkyl, Acyl oder Aryl) tragen können.

Bevorzugt sind die funktionellen Gruppe Fu ausgewählt unter -OH, -SH, -Cl, -Br, -COOR¹⁵, -O-C(=O)R¹⁶, -O-C(=O)-OR¹⁵, -O-C(=O)-NR¹⁶R¹⁷, -NR¹⁷-C(=O)-R¹⁶, -NR¹⁷-C(=O)-OR¹⁵, -NR¹⁶-C(=O)-NR¹⁷R¹⁸,
worin
- R¹⁵: für Alkyl, Cycloalkyl, Heterocycloalkyl, Aryl oder Hetaryl steht,
- R¹⁶, R¹⁷ und R¹⁸: unabhängig voneinander für Wasserstoff, Alkyl, Cycloalkyl, Heterocycloalkyl, Aryl oder Hetaryl stehen.

Lediglich zur Veranschaulichung werden im Folgenden einige geeignete funktionalisierte Olefine aufgeführt:

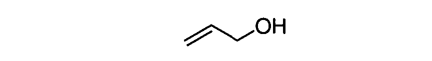

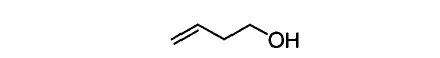

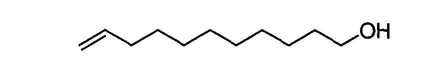

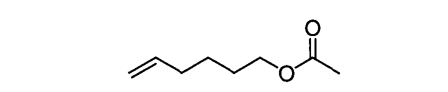

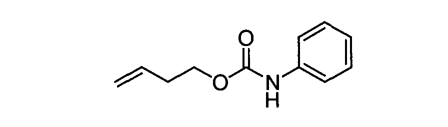

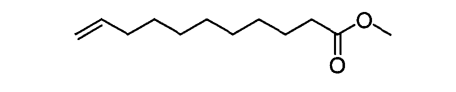

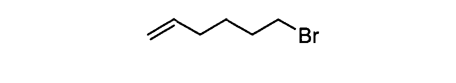

Vorzugsweise wird in dem erfindungsgemäßen Hydroformylierungsverfahren ein großtechnisch zugängiges Olefingemisch eingesetzt, das insbesondere wenigstens ein internes lineares Olefin enthält. Dazu zählen z. B. die durch gezielte Ethen-Oligomerisierung in Gegenwart von Alkylaluminiumkatalysatoren erhaltenen Ziegler-Olefine. Dabei handelt es sich im Wesentlichen um unverzweigte Olefine mit endständiger Doppelbindung und gerader Kohlenstoffatomanzahl. Dazu zählen weiterhin die durch Ethen-Oligomerisierung in Gegenwart verschiedener Katalysatorsysteme erhaltenen Olefine, z. B. die in Gegenwart von Alkylaluminiumchlorid/Titantetrachlorid-Katalysatoren erhaltenen, überwiegend linearen α-Olefine und die in Gegenwart von Nickel-Phosphinkomplex-Katalysatoren nach dem Shell Higher Olefin Process (SHOP) erhaltenen α-Olefine. Geeignete technisch zugängige Olefingemische werden weiterhin bei der Paraffin-Dehydrierung entsprechender Erdölfraktionen, z. B. der so genannten Petroleum- oder Dieselölfraktionen, erhalten. Zur Überführung von Paraffinen, vorwiegend von n-Paraffinen in Olefine, werden im Wesentlichen drei Verfahren eingesetzt:
- thermisches Cracken (Steamcracken),
- katalytisches Dehydrieren und
- chemisches Dehydrieren durch Chlorieren und Dehydrochlorieren.

Dabei führt das thermische Cracken überwiegend zu α-Olefinen, während die anderen Varianten Olefingemische ergeben, die im Allgemeinen auch größere Anteile an Olefinen mit innenständiger Doppelbindung aufweisen. Geeignete Olefingemische sind weiterhin die bei Metathese- bzw. Telomerisationsreaktionen erhaltenen Olefine. Dazu zählen z. B. die Olefine aus dem Phillips-Triolefin-Prozess, einem modifizierten SHOP-Prozess aus Ethylen-Oligomerisierung, Doppelbindungs-Isomerisierung und anschließender Metathese (Ethenolyse).

Geeignete in dem erfindungsgemäßen Hydroformylierungsverfahren einsetzbare technische Olefingemische sind weiterhin ausgewählt unter Dibutenen, Tributenen, Tetrabutenen, Dipropenen, Tripropenen, Tetrapropenen, Mischungen von Butenisomeren, insbesondere Raffinat II, Dimerbutenen, Dihexenen, Dimeren und Oligomeren aus dem Dimersol®-Prozess von IFP, Octolprozess® von Hüls, Polygasprozess etc.

Bevorzugt ist ein Verfahren, das dadurch gekennzeichnet ist, dass der Hydroformylierungskatalysator in situ hergestellt wird, wobei man mindestens einen erfindungsgemäß einsetzbaren Liganden, eine Verbindung oder einen Komplex eines Metalls der VIII. Nebengruppe und gegebenenfalls ein Aktivierungsmittel in einem inerten Lösungsmittel unter den Hydroformylierungsbedingungen zur Reaktion bringt.

Die Hydroformylierungsreaktion kann kontinuierlich, semikontinuierlich oder diskontinuierlich erfolgen.

Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben.

Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben. Im Allgemeinen wird für das erfindungsgemäße Verfahren ein Autoklav verwendet, der gewünschtenfalls mit einer Rührvorrichtung und einer Innenauskleidung versehen sein kann.

Die Zusammensetzung des im erfindungsgemäßen Verfahren eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 70:30, bevorzugt etwa 40:60 bis 60:40. Insbesondere bevorzugt wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 20 bis 180 °C, bevorzugt etwa 50 bis 150 °C. Im Allgemeinen liegt der Druck in einem Bereich von etwa 1 bis 700 bar, bevorzugt 1 bis 600 bar, insbesondere 1 bis 300 bar. Der Reaktionsdruck kann in Abhängigkeit von der Aktivität des eingesetzten erfindungsgemäßen Hydroformylierungskatalysators variiert werden. Im Allgemeinen erlauben die erfindungsgemäßen Katalysatoren auf Basis von phosphorhaltigen Verbindungen eine Umsetzung in einem Bereich niedriger Drücke, wie etwa im Bereich von 1 bis 100 bar.

Die erfindungsgemäß eingesetzten und die erfindungsgemäßen Hydroformylierungskatalysatoren lassen sich nach üblichen, dem Fachmann bekannten Verfahren vom Austrag der Hydroformylierungsreaktion abtrennen und können im Allgemeinen erneut für die Hydroformylierung eingesetzt werden.

Die zuvor beschriebenen Katalysatoren können auch in geeigneter Weise, z. B. durch Anbindung über als Ankergruppen geeignete funktionelle Gruppen, Adsorption, Pfropfung, etc. an einen geeigneten Träger, z. B. aus Glas, Kieselgel, Kunstharzen, Polymeren etc., immobilisiert werden. Sie eignen sich dann auch für einen Einsatz als Festphasenkatalysatoren.

Die Hydroformylierungsaktivität von Katalysatoren auf Basis der zuvor beschriebenen Liganden ist überraschenderweise in der Regel höher als die Isomerisierungsaktivität bezüglich der Bildung innenständiger Doppelbindungen. Vorteilhafterweise zeigen die erfindungsgemäßen und die erfindungsgemäß eingesetzten Katalysatoren bei der Hydroformylierung von α-Olefinen eine hohe Selektivität zugunsten der α-Aldehyde bzw. -Alkohole. Weiterhin eignen sich die erfindungsgemäßen Katalysatoren auch zur Hydroformylierung einer Vielzahl substituierter Olefine, die ansonsten einer Hydroformylierung nicht leicht zugängig sind. Weiterhin weisen die Katalysatoren im Allgemeinen eine hohe Stabilität unter den Hydroformylierungsbedingungen auf, so dass mit Ihnen in der Regel längere Katalysatorstandzeiten erzielt werden, als mit aus dem Stand der Technik bekannten Katalysatoren auf Basis herkömmlicher Chelatliganden. Vorteilhafterweise zeigen die erfindungsgemäßen und erfindungsgemäß eingesetzten Katalysatoren weiterhin eine hohe Aktivität, so dass in der Regel die entsprechenden Aldehyde, bzw. Alkohole in guten Ausbeuten erhalten werden. Bei der Hydroformylierung von α-Olefinen sowie von innenständigen, linearen Olefinen zeigen Sie zudem eine sehr geringe Selektivität zum Hydrierprodukt des eingesetzten Olefins.

Ein weiterer Gegenstand der Erfindung ist ein Verfahren zur Herstellung von 2-Propylheptanol, bei dem man
a) Buten oder ein Buten enthaltendes C₄-Kohlenwasserstoffgemisch in Gegenwart eines Katalysators, wie zuvor definiert, mit Kohlenmonoxid und Wasserstoff unter Erhalt eines n-Valeraldehyd enthaltenden Hydroformylierungsprodukts hydroformyliert,
b) gegebenenfalls das Hydroformylierungsprodukt einer Auftrennung unter Erhalt einer an n-Valeraldehyd angereicherten Fraktion unterzieht,
c) das in Schritt a) erhaltene Hydroformylierungsprodukt oder die in Schritt b) erhaltene an n-Valeraldehyd angereicherte Fraktion einer Aldolkondensation unterzieht,
d) die Produkte der Aldolkondensation mit Wasserstoff katalytisch zu Alkoholen hydriert, und
e) gegebenenfalls die Hydrierprodukte einer Auftrennung unter Erhalt einer an 2-Propylheptanol angereicherten Fraktion unterzieht.

a) Hydroformylierung
   Als Einsatzmaterial für die Hydroformylierung eignet sich sowohl im Wesentlichen reines 1-Buten als auch Gemische von 1-Buten mit 2-Buten und technisch erhältliche C₄-Kohlenwasserstoffströme, die 1-Buten und/oder 2 Buten enthalten. Vorzugsweise eignen sich C₄-Schnitte, die in großen Mengen aus FCC-Anlagen und aus Steamcrackern zur Verfügung stehen. Diese bestehen im Wesentlichen aus einem Gemisch der isomeren Butene und Butan.
   Als Einsatzmaterial geeignete C₄-Kohlenwasserstoffströme enthalten z. B. 50 bis 99, vorzugsweise 60 bis 90 Mol-% Butene und 1 bis 50, vorzugsweise 10 bis 40 Mol-% Butane. Vorzugsweise umfasst die Butenfraktion 40 bis 60 Mol-% 1-Buten, 20 bis 30 Mol-% 2-Buten und weniger als 5 Mol-%, insbesondere weniger als 3 Mol-% Isobuten (bezogen auf die Butenfraktion). Als besonders bevorzugter Einsatzstoff wird das so genannte Raffinat II verwendet, bei dem es sich um einen Isobuten-abgereicherten C₄-Schnitt aus einer FCC-Anlage oder einen Steamcracker handelt.
   Hydroformylierungskatalysatoren auf Basis der erfindungsgemäß eingesetzten Phosphorchelatverbindungen als Liganden weisen vorteilhafterweise eine hohe n-Selektivität, auch beim Einsatz von 2-Buten und 2-butenhaltigen Kohlenwasserstoffgemischen als Einsatzmaterial auf. Somit können in dem erfindungsgemäßen Verfahren auch solche Einsatzstoffe wirtschaftlich eingesetzt werden, da der angestrebte n-Valeraldehyd in guten Ausbeuten resultiert.
b) Auftrennung
   Nach einer geeigneten Verfahrensvariante wird die in Schritt a) nach Abtrennung des Katalysatorsystems erhaltene produktangereicherte Fraktion einer weiteren Auftrennung zum Erhalt einer an n-Valeraldehyd angereicherten Fraktion unterzogen. Die Auftrennung des Hydroformylierungsprodukts in eine n-Valeraldehyd angereicherte Fraktion und eine n-Valeraldehyd abgereicherte Fraktion erfolgt nach üblichen, dem Fachmann bekannten Verfahren. Bevorzugt ist die Destillation unter Einsatz bekannter Trennapparaturen, wie Destillationskolonnen, z. B. Bodenkolonnen, die gewünschtenfalls mit Glocken, Siebplatten, Siebböden, Ventilen etc. ausgerüstet sein können, Verdampfer, wie Dünnschichtverdampfer, Fallfilmverdampfer, Wischblattverdampfer etc.
c) Aldolkondensation
   Zwei Moleküle C₅-Aldehyd können zu α,β-ungesättigten C₁₀-Aldehyden kondensiert werden. Die Aldolkondensation erfolgt auf an sich bekannte Weise z. B. durch Einwirkung einer wässrigen Base, wie Natronlauge oder Kalilauge. Alternativ kann auch ein heterogener basischer Katalysator, wie Magnesium- und/oder Aluminiumoxid, verwendet werden (vgl. z. B. die EP-A 792 862). Dabei resultiert bei der Kondensation von zwei Molekülen n-Valeraldehyd 2-Propyl-2-heptenal. Sofern das in Schritt a) bzw. nach der Auftrennung in Schritt b) erhaltene Hydroformylierungsprodukt noch weitere C₅-Aldehyde, wie 2-Methylbutanal und gegebenenfalls 2,2-Dimethylpropanal aufweist, so untergehen diese ebenfalls eine Aldolkondensation, wobei dann die Kondensationsprodukte aller möglichen Aldehydkombinationen resultieren, beispielsweise 2-Propyl-4-methyl-2-hexenal. Ein Anteil dieser Kondensationsprodukte, z. B. von bis zu 30 Gew.-%, steht einer vorteilhaften Weiterverarbeitung zu als Weichmacheralkoholen geeigneten 2-Propylheptanol-haltigen C₁₀-Alkoholgemischen nicht entgegen.
d) Hydrierung
   Die Produkte der Aldolkondensation können mit Wasserstoff katalytisch zu C₁₀-Alkoholen, wie insbesondere 2-Propylheptanol, hydriert werden.
   Für die Hydrierung der C₁₀-Aldehyde zu den C₁₀-Alkoholen sind prinzipiell auch die Katalysatoren der Hydroformylierung zumeist bei höherer Temperatur geeignet; im Allgemeinen werden jedoch selektivere Hydrierkatalysatoren vorgezogen, die in einer separaten Hydrierstufe eingesetzt werden. Geeignete Hydrierkatalysatoren sind im Allgemeinen Übergangsmetalle, wie z. B. Cr, Mo, W, Fe, Rh, Co, Ni, Pd, Pt, Ru usw. oder deren Mischungen, die zur Erhöhung der Aktivität und Stabilität auf Trägern, wie z. B. Aktivkohle, Aluminiumoxid, Kieselgur usw. aufgebracht werden können. Zur Erhöhung der katalytischen Aktivität können Fe, Co und bevorzugt Ni, auch in Form der Raney-Katalysatoren, als Metallschwamm mit einer sehr großen Oberfläche verwendet werden. Die Hydrierung der C₁₀-Aldehyde erfolgt in Abhängigkeit von der Aktivität des Katalysators, vorzugsweise bei erhöhten Temperaturen und erhöhtem Druck. Vorzugsweise liegt die Hydriertemperatur bei etwa 80 bis 250 °C, bevorzugt liegt der Druck bei etwa 50 bis 350 bar.
   Das rohe Hydrierungsprodukt kann nach üblichen Verfahren, z. B. durch Destillation, zu den C₁₀-Alkoholen aufgearbeitet werden.
e) Auftrennung
   Gewünschtenfalls können die Hydrierprodukte einer weiteren Auftrennung unter Erhalt einer an 2-Propylheptanol angereicherten Fraktion und einer an 2-Propylheptanol abgereicherten Fraktion unterzogen werden. Diese Auftrennung kann nach üblichen, dem Fachmann bekannten Verfahren, wie z. B. durch Destillation, erfolgen.

Hydroformylierungskatalysatoren, die einen Komplex wenigstens eines Metalls der VIII. Nebengruppe des Periodensystems mit einem erfindungsgemäß einsetzbaren Liganden aufweisen, eignen sich in vorteilhafter Weise für den Einsatz in einem Verfahren zur Herstellung von 2-Propylheptanol. Dabei weisen die Katalysatoren eine hohe n-Selektivität auf, so dass sowohl beim Einsatz von im Wesentlichen reinem 1-Buten als auch beim Einsatz von 1-Buten/2-Buten-haltigen Kohlenwasserstoffgemischen, wie beispielsweise C₄-Schnitten eine gute Ausbeute an n-Valeraldehyd erhalten wird.

Ein weiterer Gegenstand der Erfindung ist die Verwendung von Katalysatoren, umfassend wenigstens einen Komplex eines Metalls der VIII. Nebengruppe mit wenigstens einem Liganden, wie zuvor beschrieben, zur Hydroformylierung, Carbonylierung und zur Hydrierung.

Die Erfindung wird anhand der folgenden, nicht einschränkenden Beispiele näher erläutert.

### Beispiel A:

### Berechnung von 6-Diphenylphosphinopyridinon-Dimeren (6-DPPon-Dimeren) zur Vorhersage ihrer Pseudochelateigenschaften

Mit Hilfe eines graphischen Molecular-Modeling-Programms werden alle möglichen H-Brücken-gebundenen Dimere von 6-DPPon und seinen Tautomeren erzeugt. Diese Dimerstrukturen werden dann mit quantenchemischen Methoden optimiert.

Als Chelatligand sind 6-DPPon-Liganden nur dann geeignet, wenn in einer berechneten Dimerstruktur der Abstand der P-Atome weniger als 5 Å beträgt.

Im Folgenden sei die berechnete Dimer-Bildung von 6-Diphenylphosphinopyridon gezeigt. Es geht aus der Berechnung hervor, dass die Wechselwirkung eines 6-Diphenylphosphinopyrid-2-ons mit des 2-Hydroxy-6-diphenylphosphinopyridins (einem Tautomer 6-Diphenylphosphinopyrid-2-ons) zu einer Anordnung führt, bei der die Phosphoratome einen Abstand von 3.8 Å aufweisen (Methode: DFT, B3-LYP, Basis TZVP (A.

Schäfer et al., J. Chem. Phys. (1994), 100, S. 5829ff)). Das System ist somit zu einer Chelatisierung und den damit bekannten Vorteilen befähigt.

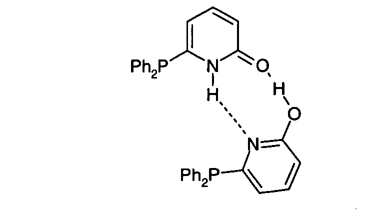

### Beispiel B:

### Kristallstrukturanalyse von [cis-PtCl₂(6-DPPon)₂]

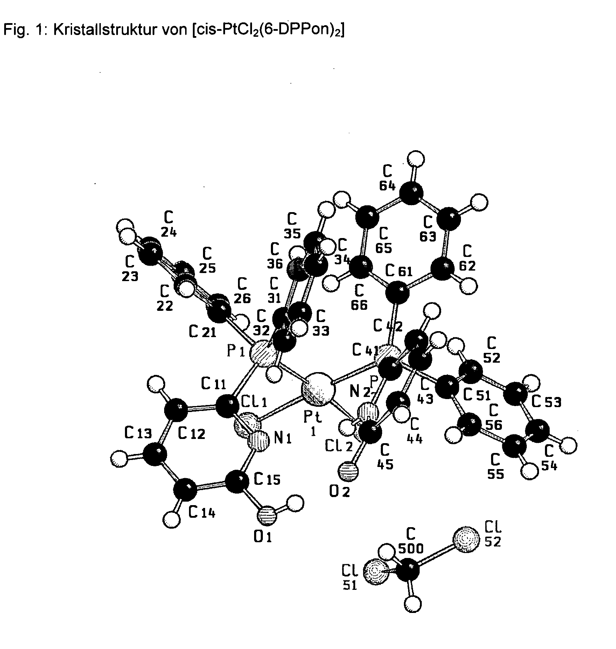

In einem Schlenkrohr wurden 68,4 mg (182 µmol, 1 eq) [cis-PtCl₂(COD)₂] in 2,5 ml CH₂Cl₂ gelöst und mit 102 mg (366 µmol, 2 eq) 6-Diphenylphosphino-1H-pyridin-2-on versetzt. Die zitronengelbe Suspension wurde mit weiteren 2,5 ml CH₂Cl₂ versetzt und die entstandene blassgelbe Lösung 5 min bei Raumtemperatur gerührt. Nach dem Abziehen des Lösungsmittels am Hochvakuum (HV) wurde der verbleibende Rückstand zweimal in je 5 ml Pentan suspendiert, das überstehende Lösungsmittel abpipettiert und der weiße Feststoff am HV getrocknet. Geeignete Kristalle für ein Kristallstrukturanalyse wurden aus einer Lösung von 20 mg [cis-PtCl₂(6-DPPon)₂] in 1 ml CH₂Cl₂ gewonnen. Figur 1 zeigt eine Ball-and-Stick-Ansicht der ermittelten Struktur.

**Tabelle 1:**

| Daten der Röntgenstrukturanalyse | |
|---|---|
| Summenformel | C₃₅ H₃₀ Cl₄ N₂ O₂ P₂ Pt |
| Molmasse | 909.44 |
| Temperatur | 100(2) K |
| Wellenlänge | 0.71073 A |
| Kristallsystem, Raumgruppe | Monoclinic, P 21/a |
| Gitterkonstanten | a = 16.8292(3) Å alpha = 90° |
| | b = 11.1616(2) Å beta = 101.7809(11)° |
| | c = 18.6043(3) Å gamma = 90° |
| Volume | 3421.03(10) Å³ |
| Z, Dichte (berechnet) | 4, 1.766 Mg/m^3 |
| Absorptionskoefficient | 4.543 mm⁻¹ |
| F(000) | 1784 |
| Kristallgröße | 0.43 x 0.3 x 0.25 mm |
| gemessener Theta-Bereich | 2.89 to 27.50° |
| Indexgrenzen | -21<=h<=21, -14<=k<=14, -14<=I<=24 |
| gemessene/unabhängige Reflexe | 19764 / 7783 [R(int) = 0.0313] |
| Absorptionskorrektur | Semi-empirisch |
| Max. and min. Transmission | 0.294 and 0.249 |
| Strukturverfeinerung | kleinste Quadrate gegen F² |
| Daten / restraints / Parameter | 7783 / 0 / 423 |
| Goodness-of-fit on F^2 | 1.059 |
| R-Werte [I>2σ(I)] | R1 = 0.0205, wR2 = 0.0478 |
| R-Werte (all data) | R1 = 0.0250, wR2 = 0.0496 |
| max/min Restelektronendichte | 0.926 und -1.475 e Å³ |

**Tabelle 2:**

| Atomkoordination und thermische Parameter | | | | |
|---|---|---|---|---|
| | x | y | z | U(eq) |
| C(11) | 4628(1) | 111(2) | 8359(1) | 18(1) |
| C(12) | 4100(2) | 888(3) | 8588(2) | 26(1) |
| C(13) | 3794(2) | 560(3) | 9208(2) | 33(1) |
| C(14) | 4037(2) | -494(3) | 9555(2) | 32(1) |
| C(15) | 4595(2) | -1211(3) | 9295(2) | 26(1) |
| C(21) | 4594(1) | 1660(2) | 7075(1) | 16(1) |
| C(22) | 4806(2) | 2766(2) | 7407(2) | 22(1) |
| C(23) | 4465(2) | 3813(2) | 7085(2) | 26(1) |
| C(24) | 3904(2) | 3760(2) | 6423(2) | 24(1) |
| C(25) | 3693(2) | 2670(2) | 6086(1) | 25(1) |
| C(26) | 4034(2) | 1620(2) | 6407(1) | 21(1) |
| C(31) | 6094(1) | 725(2) | 7866(1) | 15(1) |
| C(32) | 6480(1) | 508(2) | 8588(1) | 19(1) |
| C(33) | 7287(2) | 843(2) | 8839(2) | 24(1) |
| C(34) | 7715(2) | 1378(2) | 8362(2) | 26(1) |
| C(35) | 7335(2) | 1603(2) | 7640(2) | 24(1) |
| C(36) | 6522(2) | 1294(2) | 7392(1) | 18(1) |
| C(41) | 6646(1) | -1924(2) | 7649(1) | 15(1) |
| C(42) | 7460(2) | -1791(2) | 7753(1) | 20(1) |
| C(43) | 7936(2) | -1990(3) | 8466(2) | 25(1) |
| C(44) | 7582(2) | -2311 (2) | 9033(2) | 26(1) |
| C(45) | 6727(2) | -2440(2) | 8939(1) | 22(1) |
| C(51) | 6061(1) | -3465(2) | 6465(1) | 16(1) |
| C(52) | 5880(2) | -3750(2) | 5718(1) | 21(1) |
| C(53) | 5961 (2) | -4912(3) | 5485(2) | 25(1) |
| C(54) | 6225(2) | -5805(3) | 5993(2) | 29(1) |
| C(55) | 6406(2) | -5537(2) | 6738(2) | 28(1) |
| C(56) | 6323(2) | -4371 (2) | 6975(1) | 21(1) |
| C(61) | 6332(1) | -1021(2) | 6108(1) | 15(1) |
| C(62) | 7058(1) | -1329(2) | 5893(1) | 17(1) |
| C(63) | 7324(2) | -632(2) | 5367(1) | 21(1) |
| C(64) | 6881 (2) | 347(3) | 5053(1) | 23(1) |
| C(65) | 6156(2) | 641 (2) | 5256(1) | 22(1) |
| C(66) | 5878(2) | -51 (2) | 5776(1) | 18(1) |
| Cl(1) | 3356(1) | -935(1) | 6987(1) | 18(1) |
| Cl(2) | 4147(1) | -3078(1) | 6206(1) | 23(1) |
| N(1) | 4880(1) | -936(2) | 8699(1) | 20(1) |
| N(2) | 6299(1) | -2243(2) | 8233(1) | 18(1) |
| O(1) | 4857(1) | -2226(2) | 9650(1) | 33(1) |
| O(2) | 6354(1) | -2710(2) | 9439(1) | 29(1) |
| P(1) | 5031(1) | 300(1) | 7523(1) | 14(1) |
| P(2) | 5933(1) | -1935(1) | 6759(1) | 12(1) |
| Pt(1) | 4685(1) | -1396(1) | 6874(1) | 12(1) |
| C(500) | 5866(2) | -5480(3) | 9494(2) | 32(1) |
| Cl(51) | 4907(1) | -5174(1) | 8943(1) | 47(1) |
| Cl(52) | 6558(1) | -5969(1) | 8958(1) | 50(1) |

### Beispiele 1 - 8 (erfindungsgemäß):

### Niederdruck-Hydroformylierungen von 1-Octen

Die Hydroformylierung wurde parallel in 8 baugleichen Autoklaven durchgeführt. Dazu wurden in einem Schlenkrohr 1,8 mg (7,0 µmol) Rhodiumdicarbonylacetylacetonat in 24 ml Toluol gelöst und mit 39 mg (0,14 mmol) 6-Diphenylphosphino-1H-pyridin-2-on versetzt. Die erhaltene Lösung wurde gleichmäßig auf die 8 Autoklaven verteilt und zur Präformierung 30 Minuten bei 90 °C und unter 5 bar Synthesegas (CO/H = 1:1) gerührt. Dann wurde die Temperatur für die einzelnen Autoklaven wie in Tabelle 3 angegeben eingestellt und der Druck für alle Autoklaven einheitlich auf 10 bar CO/H₂ (1:1) erhöht. Unter diesen Bedingungen wurde pro Autoklav 0,69 g (6,2 mmol) 1-Octen über eine Schleuse zugesetzt und jede Schleuse anschließend mit 0,5 ml Toluol nachgespült. Druck und Temperatur wurden über die gesamte Reaktionszeit konstant gehalten. Nach 4 Stunden Reaktionszeit wurden die Autoklaven abgekühlt, entspannt und entleert. Die erhaltenen Reaktionslösungen wurden über Gaschromatographie (GC) analysiert. Die erhaltenen Ergebnisse sind in Tabelle 3 wiedergegeben.

**Tabelle 3:**

| Hydroformylierung von 1-Octen bei 10 bar CO/H₂ und verschiedenen Temperaturen | | | | | |
|---|---|---|---|---|---|
| Beispiel | Temperatur [°C] | 1-Octen (Umsatz) [%] | Nonanale gesamt (Ausbeute) [%] | n-Anteil^{[a]} (Selektivität) [%] | α-Anteil^{[b]}(Selektivität) [%] |
| 1 | 40 | 3 | 2 | 93 | 100 |
| 2 | 50 | 10 | 9 | 95 | 100 |
| 3 | 65 | 56 | 53 | 97 | 100 |
| 4 | 80 | 95 | 85 | 96 | 100 |
| 5 | 95 | 98 | 85 | 96 | 100 |
| 6 | 110 | 96 | 82 | 93 | 100 |
| 7 | 125 | 90 | 71 | 89 | 100 |
| 8 | 140 | 81 | 34 | 83 | 100 |

| | | | | | |
|---|---|---|---|---|---|
| [a] Anteil n-Nonanal | | | | | |
| [b] n-Nonanal + 2-Methyloctanal | | | | | |

### Vergleichsbeispiele 1 - 8

### Niederdruck-Hydroformylierungen von 1-Octen

Die Hydroformylierung wurde analog zu den Beispielen 1 - 8 parallel in 8 baugleichen Autoklaven durchgeführt. Dazu wurden in einem Schlenkrohr 1,8 mg (7,0 µmol) Rhodiumdicarbonylacetylacetonat in 24 ml Toluol gelöst und mit 37 mg (0,14 mmol) Triphenylphosphin versetzt. Die erhaltene Lösung wurde gleichmäßig auf die 8 Autoklaven verteilt und zur Präformierung 30 Minuten bei 90 °C und unter 5 bar Synthesegas (CO/H₂ = 1:1) gerührt. Dann wurde die Temperatur für die einzelnen Autoklaven wie in Tabelle 4 angegeben eingestellt und der Druck für alle Autoklaven einheitlich auf 10 bar CO/H₂ (1:1) erhöht. Unter diesen Bedingungen wurde pro Autoklav 0,69 g (6,2 mmol) 1-Octen über eine Schleuse zugesetzt und jede Schleuse anschließend mit 0,5 ml Toluol nachgespült. Druck und Temperatur wurden über die gesamte Reaktionszeit konstant gehalten. Nach 4 Stunden Reaktionszeit wurden die Autoklaven abgekühlt, entspannt und entleert. Die erhaltenen Reaktionslösungen wurden über Gaschromatographie analysiert. Die erhaltenen Ergebnisse sind in Tabelle 4 wiedergegeben.

**Tabelle 4:**

| Hydroformylierung von 1-Octen mit Rhodium/Triphenylphosphin als Katalysator bei 10 bar CO/H₂ und verschiedenen Temperaturen | | | | | |
|---|---|---|---|---|---|
| Vergleichsbeispiel | Temperatur [°C] | 1-Octen (Umsatz) [%] | Nonanale gesamt (Ausbeute) [%] | n-Anteil^{[a]}(Selektivität) [%] | α-Anteil^{[b]} (Selektivität) [%] |
| 1 | 40 | 16 | 4 | 77 | 100 |
| 2 | 50 | 17 | 6 | 76 | 100 |
| 3 | 65 | 22 | 22 | 72 | 100 |
| 4 | 80 | 98 | 89 | 73 | 100 |
| 5 | 95 | 98 | 84 | 70 | 98 |
| 6 | 110 | 99 | 67 | 61 | 94 |
| 7 | 125 | 97 | 44 | 56 | 93 |
| 8 | 140 | 94 | 12 | 62 | 97 |

| | | | | | |
|---|---|---|---|---|---|
| [a] Anteil n-Nonanal | | | | | |
| [b] n-Nonanal + 2-Methyloctanal | | | | | |

### Allgemeine Versuchsbeschreibung zur Durchführung diskontinuierlicher Hydroformylierungsversuche (Beispiele 9 - 11)

Rhodiumvorläufer, Ligand und Lösungsmittel wurden unter Stickstoff-Inertgas in einem Schlenkrohr gemischt. Die erhaltene Lösung wurde in einen mit CO/H₂ (1:1) gespülten 70 ml oder 100 ml Autoklaven überführt. Es wurden bei Raumtemperatur 5 bar CO/H₂ (1:1) aufgepresst. Unter kräftigem Rühren mit einem Begasungsrührer wurde das Reaktionsgemisch innerhalb von 30 Minuten auf die gewünschte Temperatur erhitzt. Über eine Schleuse wurde dann das eingesetzte Olefin mit CO/H₂-Überdruck in den Autoklaven gepresst. Darauf wurde sofort durch Aufpressen von CO/H₂ (1:1) der gewünschte Reaktionsdruck eingestellt. Während der Reaktion wurde der Druck im Reaktor über einen Druckregler konstant gehalten. Nach der Reaktionszeit wurde der Autoklav abgekühlt, entspannt und entleert. Eine Analyse des Reaktionsgemischs erfolgte mittels Gaschromatographie.

### Beispiel 9

### Niederdruck-Hydroformylierung von 1-Octen:

Ausgehend von 7,4 mg (29 µmol) Rhodiumdicarbonylacetylacetonat, 162 mg (0,58 mmol) 6-Diphenylphosphino-1H-pyridin-2-on, 25,6 g (228 mmol) 1-Octen und 25 g Toluol erhielt man nach 3 Stunden Reaktionszeit bei 100 °C und 10 bar CO/H₂ gemäß der allgemeinen Versuchsdurchführung einen Umsatz von 1-Octen von 100%. Die Ausbeute an Nonanalen betrug 91 %, die Selektivität zu n-Nonanal (n-Anteil) betrug 97% und die Selektivität zu n-Nonanal und 2-Methyloctanal (α-Anteil) betrug 100%.

### Beispiel 10

### Niederdruck-Hydroformylierung von 2-Octen:

Ausgehend von 7,3 mg (28 µmol) Rhodiumdicarbonylacetylacetonat, 162 mg (0,58 mmol) 6-Diphenylphosphino-1H-pyridin-2-on, 25,4 g (226 mmol) 2-Octen (Verhältnis cis:trans = 80:20) und 25 g Toluol erhielt man nach 3 Stunden Reaktionszeit bei 90 °C und 10 bar CO/H₂ gemäß der allgemeinen Versuchsdurchführung einen Umsatz von 2-Octen von 100%. Die Ausbeute an Nonanalen betrug 95%, die Selektivität zu n-Nonanal (n-Anteil) betrug 5% und die Selektivität zu n-Nonanal und 2-Methyloctanal (α-Anteil) betrug 57%.

### Beispiel 11

### Niederdruck-Hydroformylierung von 1-Buten:

Ausgehend von 1,9 mg (7,4 µmol) Rhodiumdicarbonylacetylacetonat, 39 mg (0,14 mmol) 6-Diphenylphosphino-1H-pyridin-2-on, 6,5 g (37 mmol 1-Buten) einer Mischung aus 32% 1-Buten und 68% Isobutan und 6,0 g Toluol erhielt man nach 4 Stunden Reaktionszeit bei 90 °C und 16 bar Gesamtdruck gemäß der allgemeinen Versuchsdurchführung einen Umsatz von 1-Buten von 100%. Die Ausbeute an Aldehyden betrug 100% und die Selektivität zu n-Valeraldehyd (n-Anteil) 97%.

### Beispiel 12

### Hydroformylierung funktionalisierter Olefine gemäß Tabelle 5 mit 6-Diphenylphosphino-1H-pyridin-2-on (6-DPPon) als Ligand

1,8 mg (6,98 µmol, 1 eq) [Rh(CO)₂acac] wurden in 10 ml Toluol gelöst und mit 39,1 mg (0,14 mmol, 20 eq) 6-Diphenylphosphinopyridon versetzt. Die orange gefärbte Lösung wurde 5 min gerührt und dann mit 6,98 mmol (1000 eq) des jeweiligen Substrates versetzt. Die Lösung wurde in den Autoklaven überführt, es wurden 10 bar CO/H₂ (1:1) aufgepresst und auf 70 °C aufgeheizt. Nach 20 h wurde die Reaktion durch Abkühlen auf Raumtemperatur und Entspannen des Autoklaven gestoppt. Die Lösung wurde mit ca. 50 ml Essigsäureethylester über wenig Kieselgel filtriert und im Vakuum eingeengt. Die Rohprodukte wurden mittels ¹H- und ¹³C-NMR-Spektroskopie analysiert.

### Vergleichsbeispiel 13:

### Hydroformylierung der Substrate gemäß Tabelle 5 mit Triphenylphosphin als Ligand

1,8 mg (6,98 µmol, 1 eq) [Rh(CO)₂acac] (acac = Acetylacetonat) wurden in 10 ml Toluol gelöst und mit 36,9 mg (0,14 mmol, 20 eq) Triphenylphosphin versetzt. Die blassgelbe Lösung wurde 5 min gerührt und dann mit 6,98 mmol (1000 eq) des jeweiligen Substrates versetzt. Die Lösung wurde in den Autoklaven überführt, es wurden 10 bar CO/H₂ (1:1) aufgepresst und auf 70 °C aufgeheizt. Nach 20 h wurde die Reaktion durch Abkühlen auf Raumtemperatur und Entspannen des Autoklaven gestoppt. Die Lösung wurde mit ca. 50 ml Essigsäureethylester über wenig Kieselgel filtriert und im Vakuum eingeengt. Die Rohprodukte wurden mittels ¹H- und ¹³C-NMR-Spektroskopie analysiert.

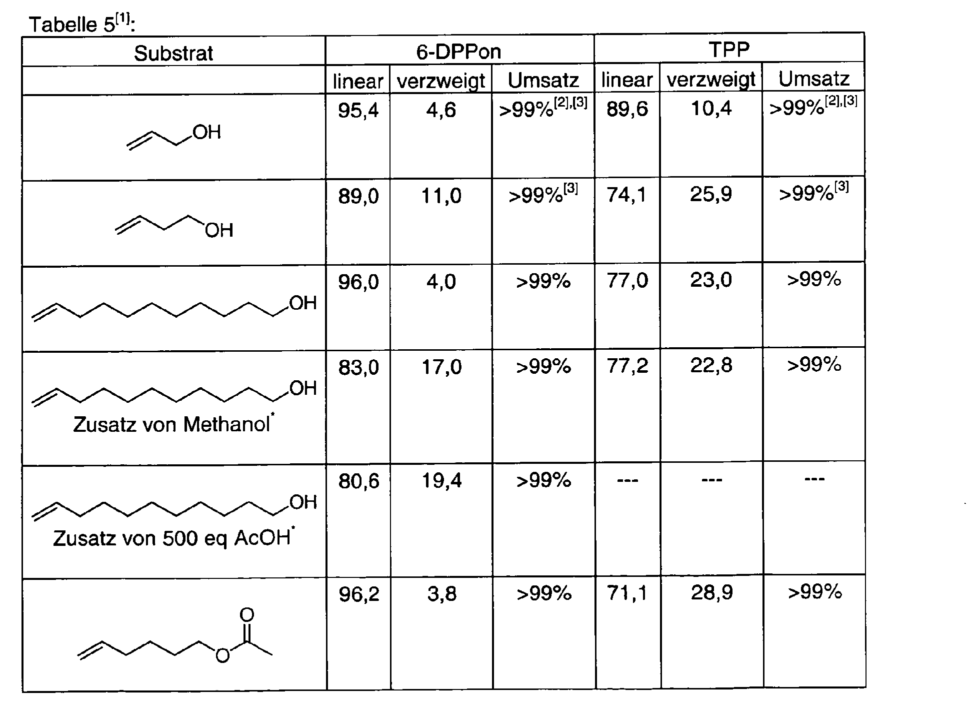

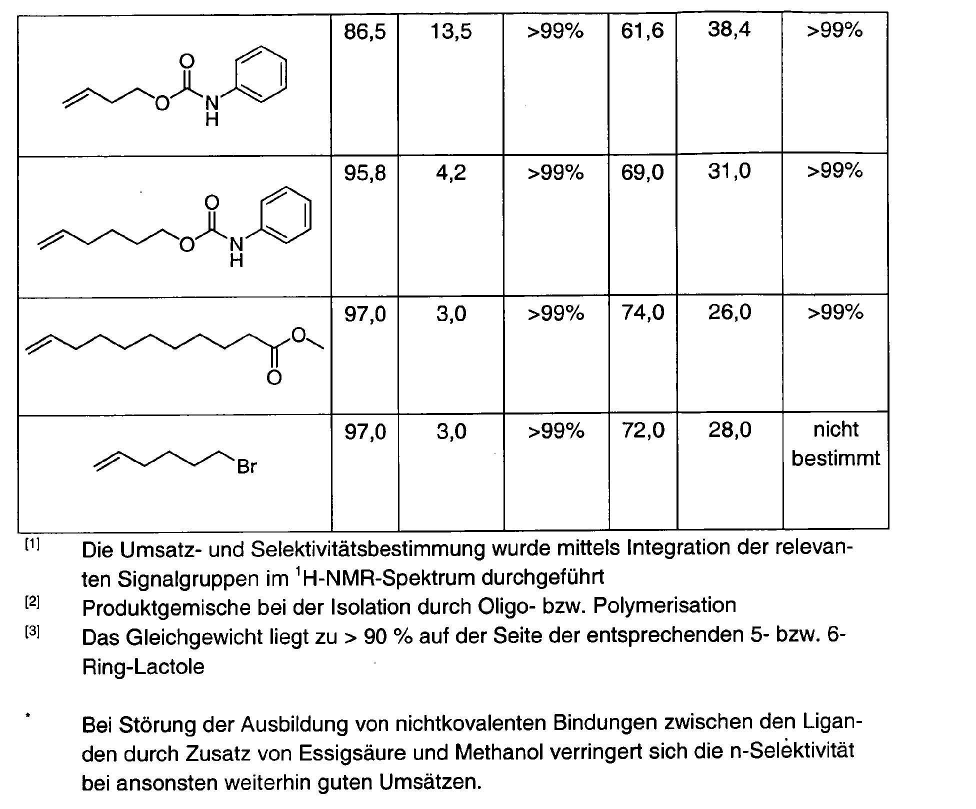

### Beispiel 14

### Darstellung von 6-(Diphenylphosphino)-2-pivalolyaminopyridin [6-DPPAP]

### 14.1 Darstellung von 6-Brom-2-aminopyridin

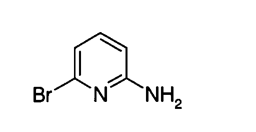

In einem Stahlautoklaven mit Glaseinsatz wurden 10,00 g 2,6-Dibrompyridin (42,2 mmol) in 50 ml konzentriertem wässrigen Ammoniak suspendiert. Der Autoklav wurde verschlossen und in einem Heizmantel 6 h auf 190 °C erhitzt (Druckanstieg auf ca. 25 bar). Nach dem Abkühlen und Entspannen des Autoklaven versetzte man den Inhalt mit 100 ml Essigsäureethylester und trennte die dabei resultierenden Phasen. Die wässrige Phase wurde zweimal mit je 100 ml Essigsäureethylester extrahiert, die vereinigten organischen Phasen wurden über Na₂SO₄ getrocknet und das Lösungsmittel im Vakuum entfernt. Der Rückstand wurde zur Abtrennung von entstandenem 2,6-Diaminopyridin in 250 ml Cyclohexan/Essigsäureethylester (1:1) gelöst, mit weiteren 250 ml Cyclohexan/Essigsäureethylester (1:1) über eine kurze Kieselgel-Säule (5 x 20 cm) filtriert und im Vakuum vom Lösungsmittel befreit. Sublimation des Rückstands bei 90 °C und 10⁻¹ mbar lieferte 6,49 g (37,5 mmol, 88,9 %) 6-Brom-2-aminopyridin als weißen Feststoff.

### 14.2 Elektrophile Route

### 14.2.1 Darstellung von 6-Brom-2-pivaloylaminopyridin

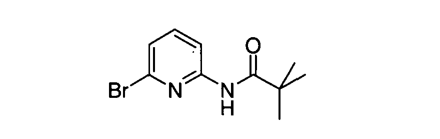

4,0 g (23,1 mmol) 6-Brom-2-aminopyridin aus Beispiel 14.1 wurden in 25 ml Dichlormethan gelöst und mit 4,1 ml (2,92 g, 28,9 mmol) Triethylamin versetzt. Nach dem Abkühlen auf 0 °C wurde eine Lösung von 3,1 ml (3,06 g, 25,4 mmol) Pivaloylchlorid in 5,0 ml Dichlormethan über einen Zeitraum von 10 min zugetropft. Man ließ das Reaktionsgemisch über Nacht auf Raumtemperatur erwärmen und goss zur Aufarbeitung auf 100 ml dest. H₂O. Die Phasen wurden getrennt, die wässrige Phase zweimal mit je 100 ml Essigsäureethylester extrahiert und die vereinigten organischen Phasen über Na₂SO₄ getrocknet. Das Lösungsmittel wurde am Vakuum entfernt, der Rückstand in Dichlormethan gelöst und über eine kurze Kieselgel-Säule (3 x 10 cm) filtriert. Nach dem Entfernen des Lösungsmittels im Vakuum erhielt man 5,73 g (22,3 mmol, 96,5 %) 6-Brom-2-pivaloylaminopyridin als weißen Feststoff.

### 14.2.2 Darstellung von 6-(Diphenylphosphino)-2-pivaloylaminopyridin [6-DPPAP]

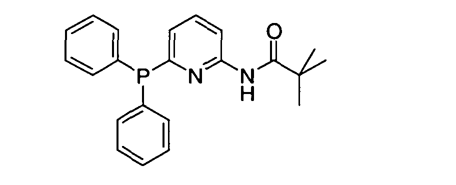

1,00 g (3,89 mmol) 6-Brom-2-pivaloylaminopyridin aus Beispiel 14.2.1 wurden in 10 ml Diethylether gelöst und bei 0 °C mit 3,64 ml (8,00 mmol) (iso-C₃H₇)MgBr (2,2M in Et₂O) versetzt. Die Suspension wurde 18 h bei 0 °C gerührt und dann mit 0,7 ml (0,86 g, 3,89 mmol) Chlordiphenylphosphin versetzt. Man ließ über Nacht auf Umgebungstemperatur erwärmen und hydrolysierte dann durch Zugabe von 10 ml dest. H₂O. Die resultierenden Phasen wurden getrennt und die wässrige Phase zweimal mit je 50 ml Essigsäureethylester extrahiert. Die organischen Phasen wurden vereinigt, über Na₂SO₄ getrocknet und das Lösungsmittel im Vakuum entfernt. Der Rückstand wurde mit Dichlormethan über eine kurze Kieselgel-Säule (2 x 10 cm) filtriert und das Lösungsmittel im Vakuum entfernt. Das erhaltene Öl wurde durch Digerieren mit Petrolether/Diethylether (40/60) kristallisiert. Die Kristalle wurden abfiltriert und mit Petrolether/Diethylether (40/60) gewaschen. Man erhielt 0,38 g (1,05 mmol, 27,0 %) 6-(Diphenylphosphino)-2-pivaloylaminopyridin als Feststoff.

³¹P-NMR (121,5 MHz, C₆D₆): δ[ppm] = -3,9

### 14.3 Nucleophile Route

### 14.3.1 Darstellung von 6-(Diphenylphosphino)-2-aminopyridin [6-DPAP]

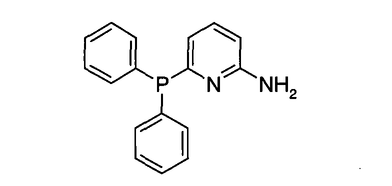

Bei -78 °C wurden 200 ml Ammoniak einkondensiert und darin innerhalb von 5 min 5,00 g Natrium (216,8 mmol) gelöst. Die resultierende dunkelblaue Lösung wurde portionsweise mit 11,70 g Triphenylphosphin (108,4 mmol) versetzt, ca. 2 h bei -78 °C gerührt und dann mit 15,00 g (86,7 mmol) 6-Brom-2-aminopyridin aus Beispiel 14.1 versetzt. Nach der Zugabe von 250 ml Toluol wurde das Kältebad entfernt und der Ammoniak über Nacht verdampft. Der Rückstand wurde mit 150 ml dest. H₂O hydrolysiert, mit 150 ml gesättigter NaCl-Lösung versetzt, die resultierenden Phasen getrennt und die wässrige Phase einmal mit 200 ml Toluol extrahiert. Die vereinigten organischen Phasen wurden über MgSO₄ getrocknet und im Vakuum eingeengt. Das Produkt wurde durch Säulenchromatographie (zuerst mit Dichlormethan zur Abtrennung von Diphenylphosphin, dann mit Dichlormethan/Essigsäureethylester 9:1) gereinigt. Erhaltene Mischfraktionen wurden durch Sublimation bei 180 °C und 10⁻² mbar gereinigt. Man erhielt 14,26 g (51,2 mmol, 59 %) 6-(Diphenylphosphino)-2-aminopyridin als weißen Feststoff.

³¹P-NMR (121,5 MHz, C₆D₆): δ[ppm] = -3,7

### 14.3.2 Darstellung von 6-(Diphenylphosphino)-2-pivaloylaminopyridin

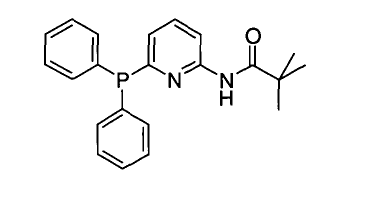

2,0 g (7,2 mmol) 6-(Diphenylphosphino)-2-aminopyridin aus Beispiel 14.3.1 wurden in 40 ml Dichlormethan gelöst und mit 1,6 ml (1,2 g, 10,8 mmol) Triethylamin und 0,16 g (0,7 mmol) 4-Dimethylaminopyridin (DMAP) versetzt. Nach dem Abkühlen auf 0 °C (H₂O/Eis) wurden 1,2 ml (1,2 g, 8,8 mmol) Pivaloylchlorid langsam zugetropft und der Ansatz für 3 h bei dieser Temperatur gehalten. Man ließ über Nacht auf Raumtemperatur erwärmen und entfernte dann das Lösungsmittel im Vakuum. Der Rückstand wurde durch Filtration über eine kurze Kieselgel-Säule (3 x 10 cm) mit Cyclohexan/Essigsäureethylester (1:1) gereinigt. Das Lösungsmittel wurde im Vakuum entfernt und das erhaltene Öl durch Digerieren mit Petrolether (40/60) kristallisiert. Der Feststoff wurde abfiltriert, mit Petrolether (40/60) gewaschen und im Vakuum getrocknet. Man erhielt 2,25 g (6,2 mmol, 86,1 %) 6-(Diphenylphosphino)-2-pivaloylaminopyridin als beigefarbenen Feststoff.

³¹P-NMR (121,5 MHz, C₆D₆): δ[ppm] = -3,9

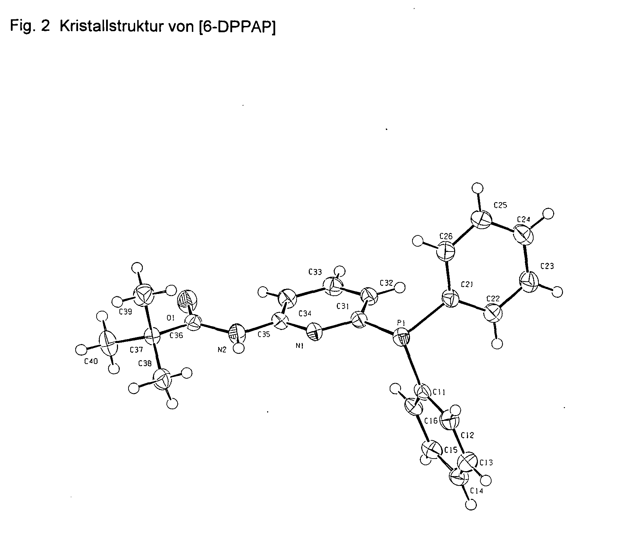

Figur 2 zeigt eine ORTEP-Darstellung der ermittelten Kristallstruktur von 6-(Diphenylphosphino)-2-pivaloylaminopyridin.

### Beispiel 15

### Darstellung von 6-(Diphenylphosphino)-2-acetylaminopyridin [6-DPAAP]

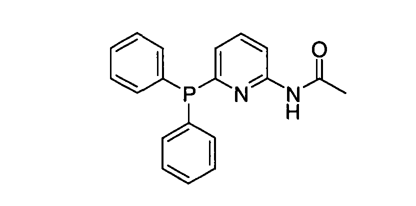

0,10 g (0,36 mmol) 6-(Diphenylphosphino)-2-aminopyridin aus Beispiel 14.3.1 wurden in 2,0 ml Dichlormethan gelöst und mit 0,05 ml (0,04 g, 0,40 mmol) Triethylamin und einer Spatelspitze DMAP versetzt. Nach der Zugabe von 0,04 ml (0,04 g, 0,40 mmol) Essigsäureanhydrid rührte man 2 h bei Raumtemperatur und entfernte dann das Lösungsmittel im Vakuum. Der Rückstand wurde säulenchromatographisch mit Cyclohexan/Essigsäureethylester (1:1) gereinigt. Man erhielt 0,074 g (0,23 mmol, 63,9 %) 6-(Diphenylphosphino)-2-acetylaminopyridin als beigefarbenen Feststoff.

³¹P-NMR (121,5 MHz, C₆D₆): δ[ppm] = -3,2

### Beispiel 16

Darstellung von 3-(Diphenylphosphino)-isochinolin-1(2*H*)-on [3-DPICon]

### 16.1 Darstellung von o-Cyanomethylbenzoesäure

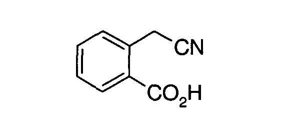

5,0 g (37,3 mmol) Phthalid und 5,0 g (76,8 mmol) Kaliumcyanid wurden fein vermörsert und unter starkem Rühren in einem offenen Rundkolben 3,5 h auf 180 °C erhitzt. Die Schmelze verfärbte sich hierbei dunkel und war nach der Reaktionszeit erstarrt. Der Feststoff wurde in 50 ml dest. H₂O gelöst und die Lösung zweimal mit je 100 ml Essigsäureethylester extrahiert. Die vereinigten organischen Phasen enthalten nicht umgesetztes Phthalid und wurden verworfen. Die wässrige Phase wurde mit 5,0 g FeSO₄·7H₂O versetzt um überschüssiges Cyanid zu binden und mit konzentriertem HCl bis zu einem pH-Wert von 2 angesäuert. Der ausgefallene Niederschlag wurde über eine mit Kieselgur gefüllte Fritte abgesaugt, mit Essigsäureethylester gewaschen und das Filtrat in einen Scheidetrichter überführt. Nach Phasentrennung wurde die wässrige Phase noch zweimal mit je 100 ml Essigsäureethylester extrahiert, die vereinigten organischen Phasen über MgSO₄ getrocknet und das Lösungsmittel im Vakuum entfernt. Man erhielt 5,34 g (33,1 mmol, 88,7 %) o-Cyanomethylbenzoesäure, die ohne weitere Reinigung für die folgende Umsetzung eingesetzt wurde.

### 16.2 Darstellung von 1,3-Dichlorisochinolin

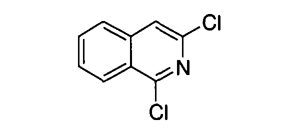

1,3 g (6,24 mmol) Phosphorpentachlorid wurden in 6 ml Phosphorylchlorid gelöst und portionsweise mit 1,0 g (6,21 mmol) *o*-Cyanomethylbenzoesäure aus Beispiel 16.1 versetzt. Nach 90 min Rühren bei Raumtemperatur war alles gelöst, und die Lösung wurde 16 h auf 70 °C erhitzt. Nach dem Abkühlen goss man vorsichtig auf 50 g Eis und versetzte mit 50 ml Essigsäureethylester. Die Phasen wurden getrennt und die wässrige Phase zweimal mit je 50 ml Essigsäure extrahiert. Die vereinigten organischen Phasen wurden mit 50 ml H₂O und 50 ml gesättigter NaHCO₃-Lösung gewaschen, über MgSO₄ getrocknet und das Lösungsmittel im Vakuum entfernt. Das braune, kristalline Rohprodukt wurde durch Filtration mit Dichlormethan/Cyclohexan (1:1) über ein kurze Kieselgel-Säule (2 x 15 cm) gereinigt. Man erhielt 1,04 g (5,25 mmol, 84,5 %) 1,3-Dichlorisochinolin.

### 16.3 Darstellung von 1-t-Butoxy-3-chlorisochinolin

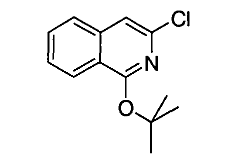

1,0 g (5,05 mmol) 1,3 Dichlorisochinolin aus Beispiel 16.2 wurde in 20 ml trockenem Toluol gelöst, mit 0,68 g (6,06 mmol) Kalium-tert.-butylat versetzt und 3 h bei 80 °C gerührt. Nach dem Abkühlen wurde mit 50 ml Dichlormethan über eine kurze Kieselgel-Säule (1 x 5 cm) filtriert und das Lösungsmittel im Vakuum entfernt. Man erhielt 1,06 g (4,50 mmol, 89,1 %) 1-*t*-Butoxy-3-chlorisochinolin.

### 16.4 Darstellung von 3-Chlorisoquinolin-1 (2H)-on

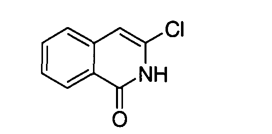

0,5 g (2,12 mmol) 1-*t*-Butoxy-3-chlorisochinolin aus Beispiel 16.3 wurden in 5,0 ml Ameisensäure gelöst und bei Raumtemperatur 20 h gerührt. Dann wurde die Lösung mit 10 ml H₂O verdünnt, der ausgefallene Feststoff über eine Glasfritte abfiltriert, mit 10 ml H₂O/Ameisensäure (2:1) gewaschen und am Hochvakuum getrocknet. Man erhielt 0,30 g (1,67 mmol, 78,8 %) 3-Chlorisoquinolin-1(2*H*)-on. Die vereinigten wässrigen Phasen wurde im Vakuum zur Trockene eingeengt und der erhaltene Feststoff durch Säulenchromatographie (Dichlormethan/Essigsäureethylester 5:1) gereinigt, worauf weitere 0,040 g (0,22 mmol, 10,4 %) 3-Chlorisoquinolin-1(2*H*)-on erhalten wurden.

### 16.5 Darstellung von 3-(Diphenylphosphino)-isochinolin-1(2H)-on [3-DPICon]

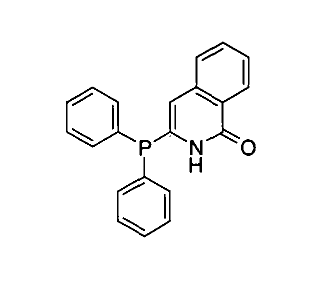

Bei -78 °C wurden 130 ml Ammoniak einkondensiert und darin innerhalb von 5 min 2,10 g Natrium (91,3 mmol) gelöst. Die dunkelblaue Lösung wurde portionsweise mit 11,70 g Triphenylphosphin (44,6 mmol) versetzt und 2 h bei -78 °C gerührt. Nach der Zugabe von 100 ml trockenem Tetrahydrofuran wurde das Kältebad entfernt und der Ammoniak innerhalb von 2 h verdampft. Nach dem Erwärmen auf Raumtemperatur wurde die orange-gefärbte Lösung portionsweise mit 4,0 g (22,3 mmol) 3-Chlorisoquinolin-1(2*H*)-on aus Beispiel 16.4 versetzt und 20 h auf 60 °C erhitzt. Nach dem Abkühlen wurde mit 50 ml dest. H₂O versetzt, die wässrige Phase dreimal mit je 100 ml Dichlormethan extrahiert, die vereinigten organischen Phasen über MgSO₄ getrocknet und im Vakuum eingeengt. Der erhaltene Feststoff wurde mit Dichlormethan digeriert, abfiltriert und getrocknet. Das Filtrat wurde eingeengt und der erhaltene Feststoff erneut mit Dichlormethan digeriert, abfiltriert und getrocknet. Die Feststoffe wurden vereinigt. Man erhielt 4,47 g (13,6 mmol, 60,9 %) 3-(Diphenylphosphino)-isochinolin-1(2*H*)-on. Das Filtrat wurde im Vakuum eingeengt und der erhaltene Feststoff durch Säulenchromatographie (Dichlormethan/Essigsäureethylester 5:1) gereinigt. Man erhielt weitere 0,216 g (0,66 mmol, 2,9 %) 3-(Diphenylphosphino)-isochinolin-1 (2H)-on.

³¹P-NMR (121,5 MHz, C₆D₆): δ[ppm] = -8,7

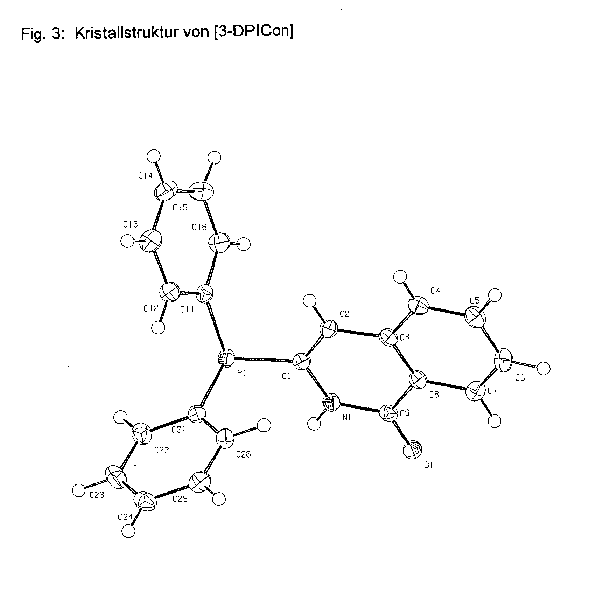

Figur 3 zeigt eine ORTEP-Darstellung der ermittelten Kristallstruktur von 3-(Diphenylphosphino)-isochinolin-1-(2H)on.

### Beispiel 17

### Darstellung von 2-Diphenylphosphinopyridin [2-DPP]

Die Synthese erfolgte zu der in analog J. Am. Chem. Soc. 1984, 106(5), 1323-32 beschriebenen Vorschrift.

### Beispiel 18

### Kristallstrukturanalyse von [cis-PtCl₂(6-DPPAP)(3-DPICon)(H₂O)]

In einem Schlenkrohr wurden 37,4 mg (100 µmol) [cis-PtCl₂(COD)], 36,2 mg (100 µmol) 6-(Diphenylphosphino)-2-pivaloylaminopyridin sowie 32,9 mg (100 µmol) 3-(Diphenylphosphino)-isochinolin-1 (2*H*)-on vorgelegt, mit 4,0 ml Toluol versetzt und durch Erhitzen auf ca. 80 °C gelöst. Beim Abkühlen fiel der Platinkomplex als weißer Feststoff aus. Das Toluol wurde abdekantiert, der Rückstand zweimal mit je 3 ml Pentan gewaschen und der weiße Feststoff am Hochvakuum getrocknet. Geeignete Kristalle für ein Kristallstrukturanalyse wurden aus einer Lösung von 10 mg [cis-PtCl₂(6-DPPAP)(3-DPICon)(H₂O)] in 1,0 ml Toluol gewonnen.

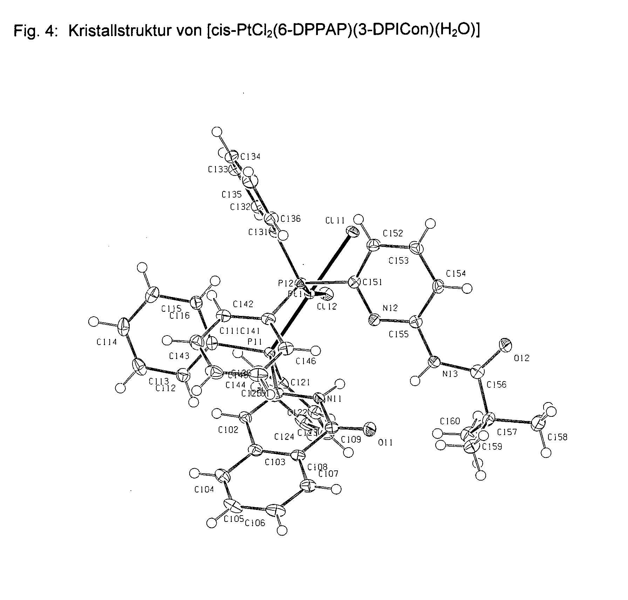

Figur 4 zeigt eine ORTEP-Darstellung der ermittelten Kristallstruktur.

### Beispiel 19

### Hydroformylierung von 1-Octen

1,8 mg (6,98 µmol) [Rh(CO)₂acac] wurden in 10 ml Toluol gelöst und mit 0,14 mmol des jeweiligen Liganden bzw. deren 1:1 Mischung (Heterodimere) versetzt. Die Lösung wurde 5 min gerührt, evtl. zur vollständigen Lösung leicht erwärmt und dann mit 1,1 ml (0,78 g, 6,98 mmol) 1-Octen versetzt. Die Lösung wurde in den Autoklaven überführt, es wurden 10 bar CO/H₂ (1:1) aufgepresst und auf 70 °C aufgeheizt. Nach 20 h wurde die Reaktion durch Abkühlen auf Raumtemperatur und Entspannen des Autoklaven gestoppt. Der Autoklavenaustrag wurde über GC und ¹H-NMR-Spektroskpie analysiert.

| Ligand(en) | Verhältnis linear:verzweigt | Umsatz [%] |
|---|---|---|
| | | |
| 2-DPP/3-DPICon | 87:13 | quantitativ |
| 2-DPAAP/3-DPICon | 95:5 | quantitativ |
| 2-DPPAP/3-DPICon | 94:6 | quantitativ |

### Beispiel 20

### Hydroformylierung verschiedener Substrate mit 2-DPPAP/3-DPICon

1,8 mg (6,98 µmol) [Rh(CO)₂acac] wurden in 10 ml Toluol gelöst und mit 0,14 mmol der beiden Liganden (1:1 Mischung) versetzt. Die Lösung wurde 5 min gerührt, evtl. zur vollständigen Lösung leicht erwärmt und dann mit 6,98 mmol des jeweiligen Substrats versetzt. Die Lösung wurde in den Autoklaven überführt, es wurden 10 bar CO/H₂ (1:1) aufgepresst und auf 70 °C aufgeheizt. Nach 20 h wurde die Reaktion durch Abkühlen auf Raumtemperatur und Entspannen des Autoklaven gestoppt. Die Lösung wurde mit ca. 50 ml Essigsäureethylester über wenig Kieselgel filtriert und im Vakuum eingeengt. Die Rohprodukte wurden mittels ¹H- und ¹³C-NMR-Spektroskopie analysiert.

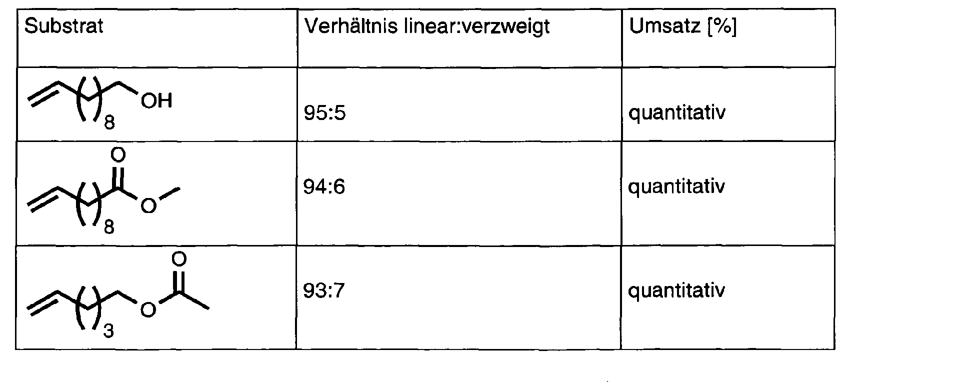

### Beispiel 21

### Quanthenchemische Rechnungen

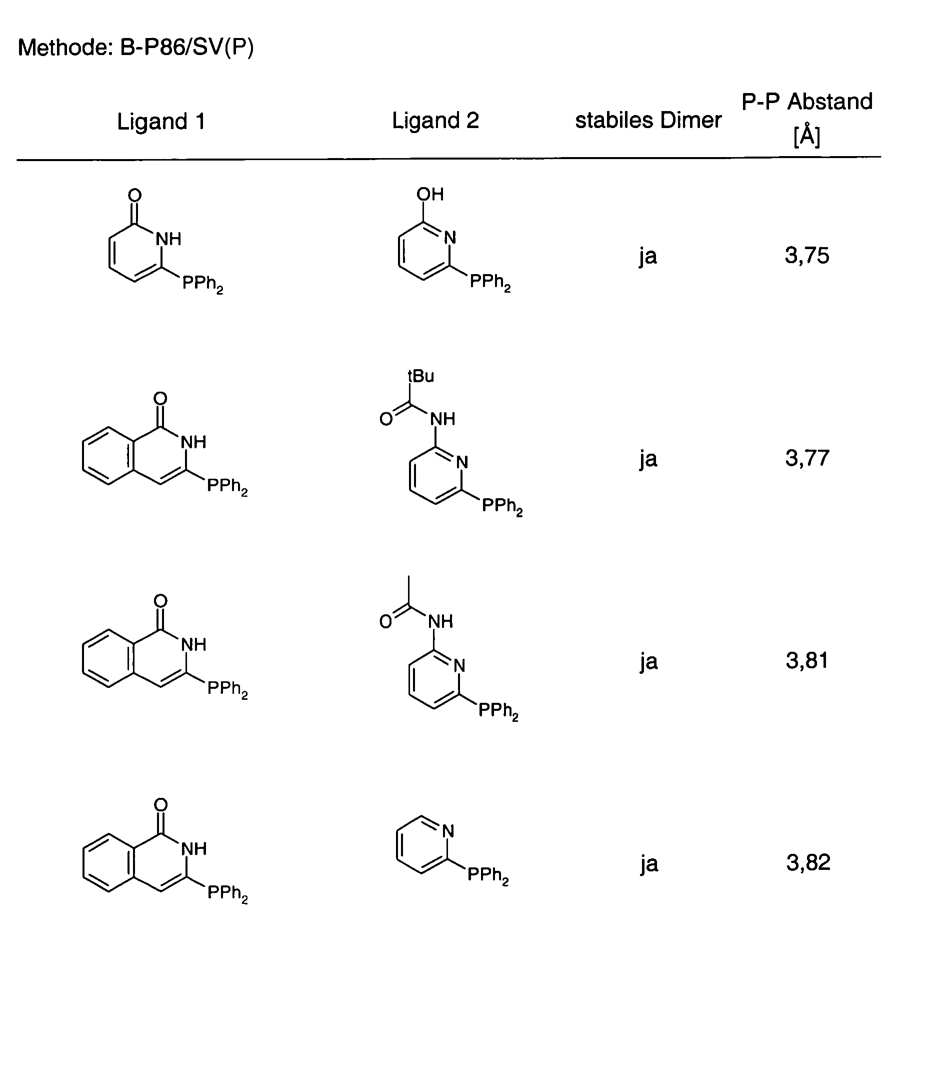

## Patentansprüche

1. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Katalysators, umfassend wenigstens einen Komplex eines Metalls der VIII. Nebengruppe des Periodensystems der Elemente mit Liganden, die jeweils eine phosphoratomhaltige Gruppe und wenigstens eine zur Ausbildung intermolekularer, nichtkovalenter Bindungen befähigte funktionelle Gruppe aufweisen, wobei der Komplex über intermolekulare nichtkovalente Bindungen dimerisierte Liganden aufweist und wobei der Abstand zwischen den Phosphoratomen der dimerisierten Liganden höchstens 5 Å beträgt.

2. Verfahren nach Anspruch 1, wobei der Abstand zwischen den Phosphoratomen der dimerisierten Liganden in einem Bereich von 2,5 bis 4,5 Å, bevorzugt 3,5 bis 4,2 Å, speziell von 3,6 bis 4,1 Å, liegt.

3. Verfahren nach einem der Ansprüche 1 oder 2, wobei die zur Ausbildung intermolekularer nichtkovalenter Bindungen befähigten funktionellen Gruppen ausgewählt sind unter Hydroxyl-, primären, sekundären und tertiären Amino-, Thiol-, Keto-, Thioketon-, Imin-, Carbonsäureester-, Carbonsäureamid-, Amidin-, Urethan-, Harnstoff-, Sulfoxid-, Sulfoximin-, Sulfonsäureamid- und Sulfonsäureestergruppen.

4. Verfahren nach einem der vorhergehenden Ansprüche, wobei die zur Ausbildung intermolekularer nichtkovalenter Bindungen befähigten funktionellen Gruppen ausgewählt sind unter Gruppen, die zur Tautomerie befähigt sind.

5. Verfahren nach einem der vorhergehenden Ansprüche, wobei die Liganden wenigstens ein Strukturelement der allgemeinen Formeln I.a oder I.b 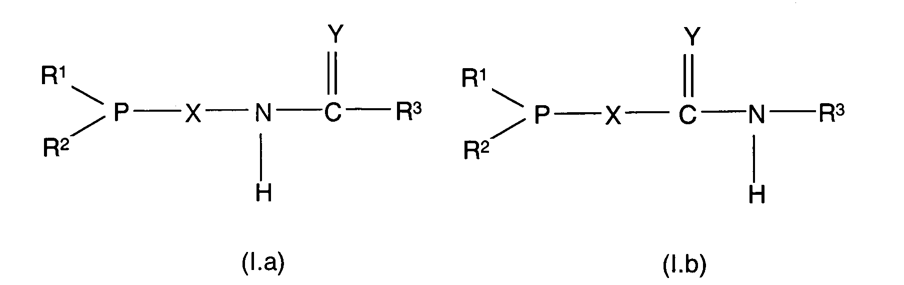 oder Tautomeren davon umfassen, worin
R¹ und R² unabhängig voneinander für Alkyl, Alkoxy, Cycloalkyl, Cycloalkoxy, Heterocycloalkyl, Heterocycloalkoxy, Aryl, Aryloxy, Hetaryl oder Hetaryloxy stehen,
R³ für Wasserstoff steht oder eine der für R¹ und R² angegebenen Bedeutungen besitzt,
X für eine zweiwertige verbrückende Gruppe mit 1 bis 5 Brückenatomen zwischen den flankierenden Bindungen steht,
Y für O, S oder NR⁴ steht, wobei R⁴ für Wasserstoff, Alkyl, Cycloalkyl, Heterocycloalkyl, Aryl oder Hetaryl steht,
wobei zwei oder mehr als zwei der Reste X und R¹ bis R⁴ gemeinsam mit dem Strukturelement der Formel I.a oder I.b, an das sie gebunden sind, für eine mono- oder polycyclische Verbindung stehen können.

6. Verfahren nach Anspruch 5, wobei in den Liganden I.a oder I.b R¹ und R² zusammen mit dem Phosphoratom, an das sie gebunden sind, für einen 5- bis 8-gliedrigen Heterocyclus stehen, der gegebenenfalls zusätzlich ein-, zwei- oder dreifach mit Cycloalkyl, Heterocycloalkyl, Aryl oder Hetaryl anelliert ist, wobei der Heterocyclus und, falls vorhanden, die anellierten Gruppen unabhängig voneinander je einen, zwei, drei oder vier Substituenten tragen können, die ausgewählt sind unter Alkyl, Cycloalkyl, Heterocycloalkyl, Aryl, Hetaryl, COOR^{c}, COO-M⁺, SO₃R^{c}, SO₃⁻M⁺, PO₃(R^{c})(R^{d})^{,} (PO₃)²⁻(M⁺)^{2,} NE⁴E⁵, (NE⁴E,⁵E⁶)⁺X^{-,} OR^{e}, SR^{e}, (CHR^{f}CH₂O)_{y}R^{e}, (CH₂NE⁴)_{y}R^{e}, (CH₂CH₂NE⁴)_{y}R^{e}, Halogen, Nitro, Acyl oder Cyano stehen, worin
R^{c} und R^{d} jeweils gleiche oder verschiedene Reste, ausgewählt unter Alkyl, Cycloalkyl, Aryl oder Hetaryl bedeuten,
R^{e}, E⁴, E⁵, E⁶ jeweils gleiche oder verschiedene Reste, ausgewählt unter Wasserstoff, Alkyl, Cycloalkyl, Acyl, Aryl oder Hetaryl bedeuten,
R^{f} für Wasserstoff, Methyl oder Ethyl steht,
M⁺ für ein Kationäquivalent steht,
X⁻ für ein Anionäquivalent steht und
y für eine ganze Zahl von 1 bis 240 steht.

7. Verfahren nach einem der vorhergehenden Ansprüche, wobei die Liganden ausgewählt sind unter Verbindungen der allgemeinen Formeln I.1 bis I.3 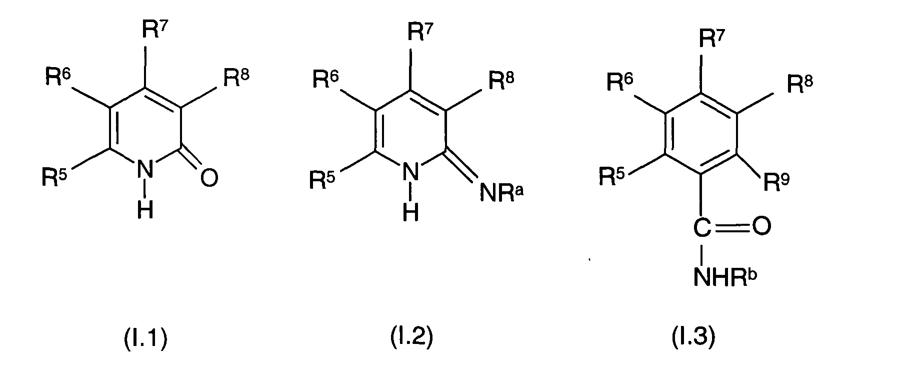 und den Tautomeren davon, worin
einer der Reste R⁵ bis R⁹ für eine Gruppe der Formel
―W'―PR¹R²
steht, worin
W' für eine Einfachbindung, ein Heteroatom, eine Heteroatom-haltige Gruppe oder eine zweiwertige verbrückende Gruppe mit 1 bis 4 Brückenatomen zwischen den flankierenden Bindungen steht,
R¹ und R² wie in einem der Ansprüche 4 oder 5 definiert sind,
die Reste R⁵ bis R⁹, die nicht für -W'-PR¹R² stehen, unabhängig voneinander für Wasserstoff, Alkyl, Cycloalkyl, Heterocycloalkyl, Aryl, Hetaryl, WCOOR^{°}, WCOO⁻M⁺, W(SO₃)R° W(SO₃)⁻M⁺, WPO₃(R^{°})(R^{P}), W(PO₃)²⁻(M⁺)₂, WNE'E², W(NE¹E²E³)⁺X⁻, WOR^{q}, WSR^{q}, (CHR^{r}CH₂O)ₓR^{q}, (CH₂NE¹)ₓR^{q}, (CH₂CH₂NE¹)ₓR^{q}, Halogen, Nitro, Acyl oder Cyano stehen,
worin
W für eine Einfachbindung, ein Heteroatom, eine Heteroatom-haltige Gruppe oder eine zweiwertige verbrückende Gruppe mit 1 bis 20 Brückenatomen steht,
R^{o} und R^{p} jeweils gleiche oder verschiedene Reste, ausgewählt unter Alkyl, Cycloalkyl, Aryl oder Hetaryl bedeuten,
R^{q}, E¹, E², E³ jeweils gleiche oder verschiedene Reste, ausgewählt unter Wasserstoff, Alkyl, Cycloalkyl, Acyl, Aryl oder Hetaryl bedeuten,
R^{r} für Wasserstoff, Methyl oder Ethyl steht,
M⁺ für ein Kationäquivalent steht,
X⁻ für ein Anionäquivalent steht und
x für eine ganze Zahl von 1 bis 240 steht,
wobei jeweils zwei benachbarte Reste R⁵, R⁶, R⁷, R⁸ und R⁹ zusammen mit den Ringkohlenstoffatomen, an die sie gebunden sind, auch für ein kondensiertes Ringsystem mit 1, 2 oder 3 weiteren Ringen stehen können, und
R^{a} und R^{b} für Wasserstoff, Alkyl, Acyl, Cycloalkyl, Heterocycloalkyl, Aryl oder Hetaryl stehen.

8. Verfahren zur Hydroformylierung von Verbindungen, die wenigstens eine ethylenisch ungesättigte Doppelbindung enthalten, durch Umsetzung mit Kohlenmonoxid und Wasserstoff in Gegenwart eines Katalysators, umfassend wenigstens einen Komplex eines Metalls der VIII. Nebengruppe des Periodensystems der Elemente mit Liganden, die ausgewählt sind unter Verbindungen der allgemeinen Formeln I.1 bis I.3. 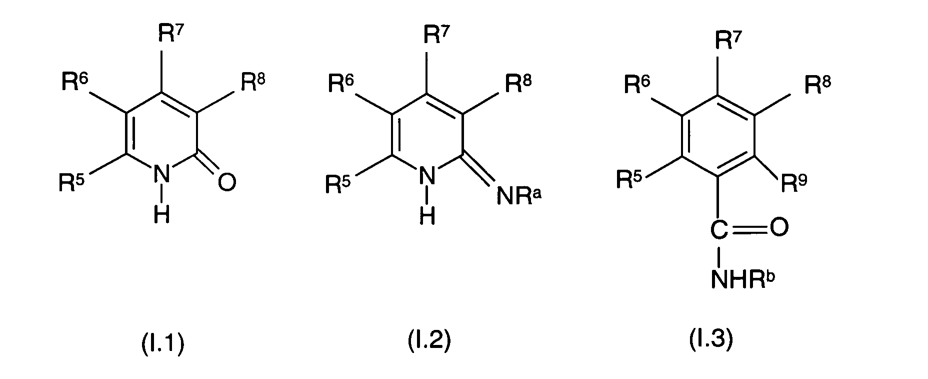 und den Tautomeren davon, worin
einer der Reste R⁵ bis R⁹ für eine Gruppe der Formel
―W'―PR¹R²
steht, worin
W' für eine Einfachbindung, ein Heteroatom, eine Heteroatom-haltige Gruppe oder eine zweiwertige verbrückende Gruppe mit 1 bis 4 Brückenatomen zwischen den flankierenden Bindungen steht,
R¹ und R² wie in einem der Ansprüche 4 oder 5 definiert sind,
die Reste R⁵ bis R⁹, die nicht für -W'-PR¹R² stehen, unabhängig voneinander für Wasserstoff, Alkyl, Cycloalkyl, Heterocycloalkyl, Aryl, Hetaryl, WCOOR^{°}, WCOO⁻M⁺, W(SO₃)R^{°}, W(SO₃)⁻M⁺, WPO₃(R^{°})(R^{P}), W(PO₃)²⁻(M⁺)₂, WNE¹E², W(NE¹E²E³)⁺X⁻, WOR^{q}, WSR^{q}, (CHR^{r}CH₂O)ₓR^{q}, (CH₂NE¹)ₓR^{q}, (CH₂CH₂NE¹)ₓR^{q}, Halogen, Nitro, Acyl oder Cyano stehen,
worin
W für eine Einfachbindung, ein Heteroatom, eine Heteroatom-haltige Gruppe oder eine zweiwertige verbrückende Gruppe mit 1 bis 20 Brückenatomen steht,
R^{o} und R^{p} jeweils gleiche oder verschiedene Reste, ausgewählt unter Alkyl, Cycloalkyl, Aryl oder Hetaryl bedeuten,
R^{q}, E¹, E², E³ jeweils gleiche oder verschiedene Reste, ausgewählt unter Wasserstoff, Alkyl, Cycloalkyl, Acyl, Aryl oder Hetaryl bedeuten,
R' für Wasserstoff, Methyl oder Ethyl steht,
M⁺ für ein Kationäquivalent steht,
X⁻ für ein Anionäquivalent steht und
x für eine ganze Zahl von 1 bis 240 steht,
wobei jeweils zwei benachbarte Reste R⁵, R⁶, R⁷, R⁸ und R⁹ zusammen mit den Ringkohlenstoffatomen, an die sie gebunden sind, auch für ein kondensiertes Ringsystem mit 1, 2 oder 3 weiteren Ringen stehen können, und
R^{a} und R^{b} für Wasserstoff, Alkyl, Acyl, Cycloalkyl, Heterocycloalkyl, Aryl oder Hetaryl stehen.

9. Verfahren nach einem der Ansprüche 6 oder 7, wobei die Liganden ausgewählt sind unter Verbindungen der allgemeinen Formeln I.i bis I.iii 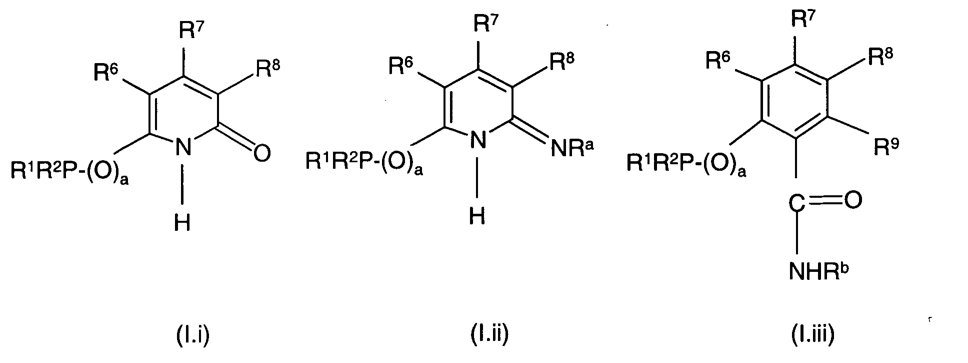 und den Tautomeren davon, worin
a für 0 oder 1 steht,
R¹ und R² wie zuvor definiert sind,
R⁶ bis R⁹ unabhängig voneinander für Wasserstoff, C₁-C₄-Alkyl, C₁-C₄-Alkoxy, Acyl, Aryl, Heteroaryl, Halogen, C₁-C₄-Alkoxycarbonyl oder Carboxylat stehen,
wobei jeweils zwei benachbarte Reste R⁶, R⁷, R⁸ und R⁹ zusammen mit den Ringkohlenstoffatomen, an die sie gebunden sind, auch für ein kondensiertes Ringsystem mit 1, 2 oder 3 weiteren Ringen stehen können, und
R^{a} und R^{b} für Wasserstoff, Alkyl, Acyl, Cycloalkyl oder Aryl stehen.

10. Verfahren nach einem der vorhergehenden Ansprüche, wobei die eingesetzten Liganden wenigstens eine Verbindung der Formeln (1) bis (4) 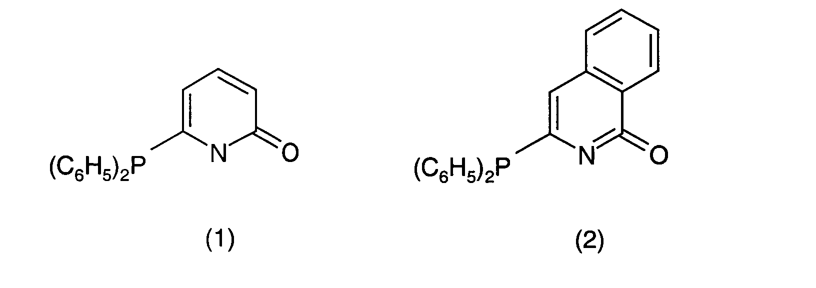 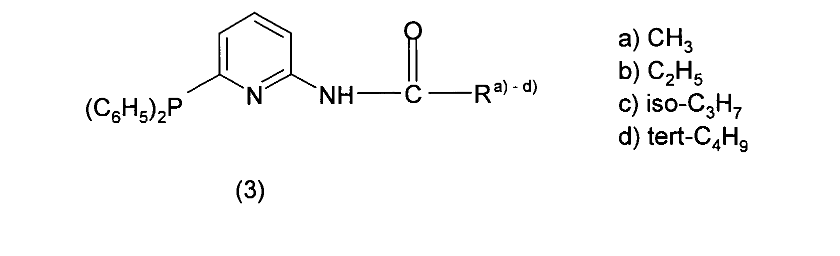 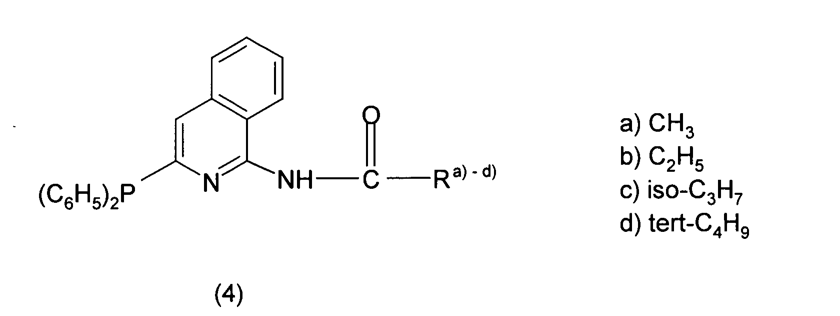 umfassen

11. Verfahren nach einem der vorhergehenden Ansprüche, wobei als Liganden eines der folgenden Ligand/Ligand-Paare (5) bis (8) eingesetzt wird

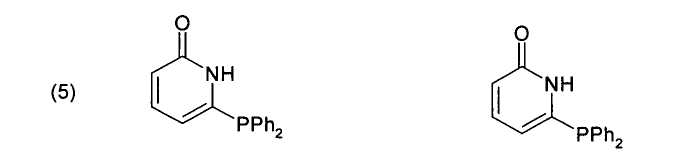

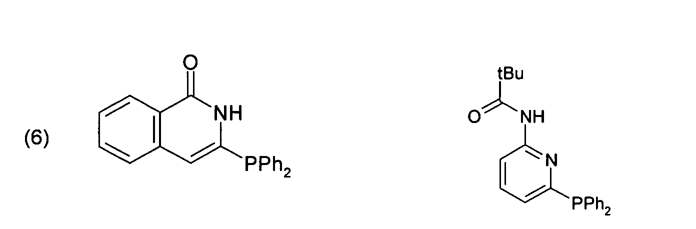

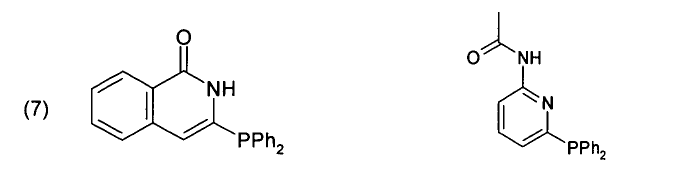

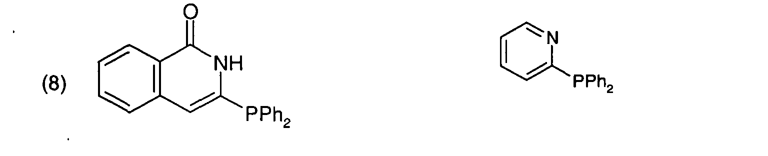

12. Katalysator, wie in einem der Ansprüche 1 bis 11 definiert.

13. Katalysator nach Anspruch 12, wobei das Metall ausgewählt ist aus Kobalt, Nickel, Rhodium, Ruthenium oder Iridium.

14. Verfahren zur Herstellung von 2-Propylheptanol, bei dem man
a) Buten oder ein Buten enthaltendes C₄-Kohlenwasserstoffgemisch in Gegenwart eines Katalysators, wie in einem der Ansprüche 12 oder 13 definiert, mit Kohlenmonoxid und Wasserstoff unter Erhalt eines n-Valeraldehyd enthaltenden Hydroformylierungsprodukts hydroformyliert,
b) gegebenenfalls das Hydroformylierungsprodukt einer Auftrennung unter Erhalt einer an n-Valeraldehyd angereicherten Fraktion unterzieht,
c) das in Schritt a) erhaltene Hydroformylierungsprodukt oder die in Schritt b) erhaltene an n-Valeraldehyd angereicherte Fraktion einer Aldolkondensation unterzieht,
d) die Produkte der Aldolkondensation mit Wasserstoff katalytisch zu Alkoholen hydriert, und
e) gegebenenfalls die Hydrierprodukte einer Auftrennung unter Erhalt einer an 2-Propylheptanol angereicherten Fraktion unterzieht.

15. Verfahren zur Herstellung eines Estergemischs, wobei man ein Alkoholgemisch, erhältlich durch ein Verfahren wie in Anspruch 14 definiert, mit wenigstens einer Säure, die ausgewählt ist unter aliphatischen Di- und Tricarbonsäuren, aromatischen Mono-, Di- und Tricarbonsäuren, Phosphorsäure und Derivaten und Mischungen davon, umsetzt.

16. Verwendung eines Katalysators, wie in einem der Ansprüche 12 oder 13 definiert, zur Hydroformylierung, Carbonylierung oder Hydrierung.
